(19) **Europäisches Patentamt** **European Patent Office** **Office européen des brevets**

(11) **EP 4 768 568 A1**

(12) **EUROPEAN PATENT APPLICATION**
published in accordance with Art. 153(4) EPC

(43) Date of publication:
01.07.2026 Bulletin 2026/27

(21) Application number: 24856532.7

(22) Date of filing: 22.08.2024

(51) International Patent Classification (IPC):
*C12N 1/12* *(2026.01)*

(52) Cooperative Patent Classification (CPC):
C12N 1/12

(86) International application number:
PCT/JP2024/029913

(87) International publication number:
WO 2025/041844 (27.02.2025 Gazette 2025/09)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 22.08.2023 JP 2023134686

(71) Applicant: Japan Science and Technology Agency
Kawaguchi-shi, Saitama 332-0012 (JP)

(72) Inventors:
• HIROOKA, Shunsuke
  Mishima-shi, Shizuoka 411-8540 (JP)
• ZHOU, Baifeng
  Mishima-shi, Shizuoka 411-8540 (JP)
• MIYAGISHIMA, Shin-ya
  Mishima-shi, Shizuoka 411-8540 (JP)

(74) Representative: Vossius & Partner
Patentanwälte Rechtsanwälte mbB
Siebertstraße 3
81675 München (DE)

(54) **UNICELLULAR ALGAE, METHOD FOR HIGH-DENSITY CULTURE OF UNICELLULAR ALGAE, AND METHOD FOR CREATING UNICELLULAR ALGAE**

(57) Unicellular algae that are capable of growing in a culture medium containing 0.1% by mass or more of sugars and having acidic conditions at a pH of 5 or less, in which a chlorophyll content of the unicellular algae after culture for 7 days under dark conditions in the presence of an organic carbon source is 1 $\mu$g/mg dry weight or more.

**EP 4 768 568 A1**

**Description**

TECHNICAL FIELD

The present invention relates to unicellular algae, a method for high-density culture of unicellular algae, and a method for producing unicellular algae.

Priority is claimed on Japanese Patent Application No. 2023-134686, filed August 22, 2023, the content of which is incorporated herein by reference.

BACKGROUND ART

In a case of industrially and massively using photosynthetic algae, it is desirable that the photosynthetic algae can be cultured while being photosynthesized in an outdoor open system using solar energy. Since solar energy is distributed broadly and thinly on the ground, a large area is required for the culture, and in order to produce a large amount, it is preferable that the culture can be performed at a high density. However, as the density of the algae increases, the sunlight is not evenly provided, and there is concern that the production of the algae may decrease.

It is known that algae belonging to the class Cyanidiophyceae grow under the conditions of acidic hot springs (high temperature and low pH). Since it is difficult for other organisms to grow under the conditions of high temperature and low pH, it is difficult for other organisms to be contaminated even in a case of being cultured in an outdoor open system. Therefore, it is considered that the algae belonging to the class Cyanidiophyceae are suitable for large-scale culture in an outdoor open system.

Among the class Cyanidiophyceae, it is known that the genus Galdieria proliferates even under heterotrophic conditions (Non-Patent Document 1). It is known that, in many photosynthetic algae, the amount of photosynthetic pigments such as chlorophyll decreases in a culture medium containing sugars. It is known that certain types of Galdieria are not likely to decrease the chlorophyll content even in a culture medium containing sugars (Non-Patent Document 1).

As the algae belonging to the class Cyanidiophyceae, the genus Cyanidioschyzon, the genus Cyanidiococcus, the genus Cyanidium, and the genus Galdieria are known. Among these, Cyanidioschyzon merolae of the genus Cyanidioschyzon is known to be haploid. On the other hand, in other algae belonging to the class Cyanidiophyceae, haploid algae have not been known, but it has recently been reported that haploid algae can be generated from diploid algae (Patent Document 1 and Non-Patent Document 2).

Citation List

Patent Documents

Patent Document 1: PCT International Publication No. WO2019/107385

Non-Patent Documents

Non-Patent Document 1: Wolfgang Gross and Claus Schnarrenberger, Heterotrophic Growth of Two Strains of the Acido-Thermophilic Red Alga Galdieria sulphuraria. Plant Cell Physiol. 36(4): 633-638 (1995).
Non-Patent Document 2: Life cycle and functional genomics of the unicellular red alga Galdieria for elucidating algal and plant evolution and industrial use. Proc Natl Acad Sci U S A. 119(41):e2210665119 (2022)

SUMMARY OF INVENTION

Technical Problem

In a case of industrially producing photosynthetic algae, it is expected that a large amount of algal cells can be efficiently obtained while suppressing the energy required for the culture, in a case where the photosynthetic algae can be cultured at a high density while using both proliferation by photosynthesis and proliferation by heterotrophic nutrition. However, it is known that, in a case where sugars for heterotrophic nutrition are added to the culture medium for the photosynthetic algae, the photosynthetic ability is reduced due to the decrease in chlorophyll, so-called "color fading" occurs. Therefore, in mixotrophic culture, the photosynthetic algae may not be cultured at a high density.

Therefore, an object of the present invention is to provide algae that can be cultured at a high density under mixotrophic conditions, a method for high-density culture of algae using the algae, and a method of producing the algae.

Solution to Problem

The present inventors have succeeded in producing algae that can efficiently proliferate in mixotrophic culture, and have completed the present invention.

The present invention includes the following aspects.

[1] A method for high-density culture of unicellular algae, the method including: performing mixotrophic culture of unicellular algae, in which the unicellular algae are algae that are capable of growing in a culture medium containing 0.1% by mass or more of sugars and having acidic conditions at a pH of 5 or less, and a chlorophyll content of the unicellular algae after culture for 7 days under dark conditions in the presence of an organic carbon source is 1 $\mu$g/mg dry weight or more.

[2] The method for high-density culture of unicellular algae according to [1], in which the unicellular algae are haploids of algae belonging to the class Cyanidiophyceae.

[3] The method for high-density culture of unicellular algae according to [1], in which the unicellular algae are homozygous diploids produced from haploids of algae belonging to the class Cyanidiophyceae.

[4] A method for producing unicellular algae, the method including: (ia) a step of producing haploid unicellular algae from diploid unicellular algae; (iia) a step of seeding the haploid unicellular algae on a solid culture medium containing an organic carbon source and culturing the seeded haploid unicellular algae under dark conditions; and (iiia) a step of collecting a green colony from colonies that have appeared on the solid culture medium.

[5] The method of producing unicellular algae according to [4], in which the unicellular algae are algae belonging to the class Cyanidiophyceae.

[6] A method for producing unicellular algae, the method including: (ib) a step of producing diploid unicellular algae from haploid unicellular algae; (iib) a step of seeding the diploid unicellular algae on a solid culture medium containing an organic carbon source and culturing the seeded diploid unicellular algae under dark conditions; and (iiib) a step of collecting a green colony from colonies that have appeared on the solid culture medium.

[7] The method of producing unicellular algae according to [6], in which the unicellular algae are algae belonging to the class Cyanidiophyceae.

[8] Unicellular algae that are capable of growing in a culture medium containing 0.1% by mass or more of sugars and having acidic conditions at a pH of 5 or less, in which a chlorophyll content of the unicellular algae after culture for 7 days under dark conditions in the presence of an organic carbon source is 1 $\mu$g/mg dry weight or more.

[9] The unicellular algae according to [8], in which the unicellular algae are haploid algae belonging to the class Cyanidiophyceae.

[10] The unicellular algae according to [8], in which the unicellular algae are homozygous diploid algae produced from haploid algae belonging to the class Cyanidiophyceae.

Advantageous Effects of Invention

An object of the present invention is to provide algae that can be cultured at a high density under mixotrophic conditions, a method for high-density culture of algae using the algae, and a method of producing the algae.

BRIEF DESCRIPTION OF DRAWINGS

[FIG. 1] A schematic of a sample adjustment method for metabolome analysis performed in Examples.

[FIG. 2] A photograph of a colony (2N) of Galdieria partita NBRC 102759 (diploid) and a colony obtained by seeding a haploid produced from the diploid algae on a glucose-containing gellan gum plate (100 mM glucose, pH 2.0) and statically culturing the seeded haploid algae at 42°C under dark conditions for about 2 weeks. The colonies N1, N5, and N6 exhibited a dark green color as compared with the colonies N2, N3, and N4.

[FIG. 3] Results of measuring the specific growth rates of colony N1 (N1 strain) and colony N2 (N2 strain), which are shown in FIG. 1, in mixotrophic culture and heterotrophic culture.

[FIG. 4] Results of measuring the vitamin C content of algal cells in mixotrophic culture and heterotrophic culture of the N1 strain and the N2 strain.

[FIG. 5] Photographs of culture solutions on the 0th day and the 20th day and a microscopic photograph of a culture solution on the 20th day in a case where the N1 strain was open-cultured in a greenhouse using an MA2 + 0.25 M glucose culture medium (pH 1.0 or pH 2.0) or a seawater + 0.25 M glucose culture medium (pH 1.0 or pH 2.0).

[FIG. 6] Growth of the N1 strain in a case where the N1 strain is open-cultured in a greenhouse using an MA2 + 0.25 M glucose culture medium (pH 1.0) and a seawater + 0.25 M glucose culture medium (pH 1.0).

[FIG. 7] Results of measuring the maximum cell density achieved by $OD_{750}$ in mixotrophic culture and heterotrophic culture of the N1 strain.

[FIG. 8] Results of measuring the dissolved oxygen concentration in mixotrophic culture and heterotrophic culture of the N1 strain.

[FIG. 9] Results of calculating the dissolved oxygen concentration per cell of the N1 strain in mixotrophic culture and heterotrophic culture of the N1 strain.

DESCRIPTION OF EMBODIMENTS

The term "includes" (comprises) means that constituent elements other than the target constituent elements may be included. The term "consists of" means that constituent elements other than the target constituent elements are not included. The term "consists essentially of" means that constituent elements other than the target constituent elements are not included in a manner whereby a special function is exhibited (such as a manner in which the effect of the invention is completely lost). In the present specification, the term "includes" (comprises) encompasses the meanings of "consists of" and "consists essentially of".

A numerical range represented by "to" refers to a range including numerical values denoted before and after "to" as a lower limit value and an upper limit value.

The cell may be isolated. The term "isolated" means a natural state or a state of being separated from other components. The "Isolated" one does not substantially include other components. The term "does not substantially include other components" means that the amount of other components included in the isolated component is negligible. The amount of other components included in the isolated component is, for example, 10% by mass or less, 5% by mass or less, 4% by mass or less, 3% by mass or less, 2% by mass or less, 1% by mass or less, 0.5% by mass or less, or 0.1% by mass or less. The cells described in the present specification can be isolated cells. The cells include unicellular algae.

The term "autotrophic conditions" refers to conditions in the light and in the absence of an organic carbon source. The culture of the algae under the autotrophic conditions is referred to as "autotrophic culture". In the autotrophic culture, the algae are cultured in the light condition (under light irradiation or under natural light) using a culture medium containing no organic carbon source (inorganic salt culture medium). In the autotrophic culture, the algae proliferate only by autotrophic proliferation through photosynthesis.

The term "heterotrophic conditions" refers to conditions in the dark and in the presence of an organic carbon source. The culture of the algae under the heterotrophic conditions is referred to as "heterotrophic culture". In the heterotrophic culture, the algae are cultured using a culture medium containing an organic carbon source. In the heterotrophic culture, the algae proliferate only by heterotrophic proliferation by assimilating the organic carbon source.

The term "mixotrophic conditions" refers to conditions in the light and in the presence of an organic carbon source. The culture of the algae under the mixotrophic conditions is referred to as "mixotrophic culture". In the mixotrophic culture, the algae are cultured in the light condition (under light irradiation or under natural light) using a culture medium containing an organic carbon source. In the mixotrophic culture, the algae proliferate both by autotrophic proliferation through photosynthesis and by heterotrophic proliferation by assimilating the organic carbon source.

The term "culture medium" refers to a composition used for culturing microorganisms such as unicellular algae. The culture medium contains a component necessary for the growth of the microorganism to be cultured. A culture medium in a solid form is referred to as a solid culture medium. A culture medium in a liquid form is referred to as a liquid culture medium.

The term "culture solution" refers to a mixture of a liquid culture medium and a microorganism, in which the microorganism is being cultured in the liquid culture medium or has been cultured.

[Unicellular algae]

A first aspect of the present invention is unicellular algae that can grow in a culture medium containing 0.1% by mass or more of sugars and having a pH of 5 or less, in which a chlorophyll content of the unicellular algae after culture for 7 days under dark conditions in the presence of an organic carbon source is 1 $\mu$g/mg dry weight or more.

(Unicellular algae)

The unicellular algae (hereinafter, also simply referred to as "algae") according to the present embodiment can grow in the presence of 0.1% by mass or more of sugars and under acidic conditions of pH 5 or less. In addition, even after culture for 7 days under dark conditions in the presence of an organic carbon source, the chlorophyll content of the algae is 1 $\mu$g/mg dry weight or more. The chlorophyll content can be calculated according to the method described in A. R. Wellburn (A. R. Wellburn, J. Plant Physiol. 144, 307-313 (1994)). In a case where the algae according to the present embodiment are cultured by a batch process, the acidity of the culture solution during the culture is usually decreased as the culture proceeds. Therefore, in a case of being cultured by a batch process, it is preferable to start the culture from a pH of 5, it is more preferable to start the culture from a pH of 2 for preventing contamination with mold, and it is still more preferable to start the culture from a pH of 1. Even in a case where the culture is performed by a continuous process and the pH of the culture solution can be controlled, it is necessary to select the pH of the culture solution in consideration of this point. In controlling the pH of the culture solution, in a case of decreasing the pH, an acidic compound such as sulfuric acid and hydrochloric acid can be used, and in a case of increasing the pH, a basic compound such as ammonium hydroxide and sodium hydroxide can be used.

In a case where the algae are cultured under the autotrophic conditions, as the density of the algae increases due to the proliferation of the algae, it becomes difficult for light to reach the lower part and the deep part of the culture tank because the light is blocked by the algal body. Therefore, the algal cells present in the lower part and the deep part of the culture tank cannot perform photosynthesis. On the other hand, in a case where the algae are cultured under the heterotrophic conditions, the growth of the algae is stopped due to the depletion of the organic carbon source.

It is considered that, under the mixotrophic conditions, the algae present in the surface layer of the culture tank can proliferate autotrophically, and the algae present in the lower part and the deep part of the culture tank can proliferate heterotrophically. However, in a case where the algae are cultured in a culture medium containing an organic carbon source, the chlorophyll content of the algae is decreased, and the photosynthetic ability may be decreased. In the algae according to the present embodiment, the chlorophyll content is maintained at 1 $\mu$g/mg dry weight or more even in a case of being cultured by heterotrophic culture. Therefore, in a case of performing mixotrophic culture, the algae according to the present embodiment can proliferate at a high density by using both the assimilation of the organic carbon source and photosynthesis.

In the algae according to the present embodiment, it is preferable that in the logarithmic growth phase under the heterotrophic conditions and the mixotrophic conditions, the growth rate under the temperature conditions of 30°C or higher and 45°C or lower is higher than the growth rate under the temperature conditions of 25°C or higher and lower than 30°C.

In the algae according to the present embodiment, the ratio of the chlorophyll content per dry weight at 12 hours after the end of the logarithmic growth phase to the chlorophyll content per dry weight at 12 hours after the start of the logarithmic growth phase under the heterotrophic conditions and the mixotrophic conditions is preferably 0.8 to 1.2, and is 0.9 to 1.1 in a case where more favorable culture is performed. It is known that, even in photosynthetic algae capable of well-known heterotrophic culture, the chlorophyll content is decreased in a case where an organic carbon source is added to the culture solution. The organic carbon source can be added to the culture solution to compensate for the fact that the algal cells cannot perform photosynthesis in the lower part and the deep part of the culture tank by high-density culture. In the algae according to the present embodiment, the chlorophyll content is maintained at 1 $\mu$g/mg dry weight or more even in a high-density state after the logarithmic growth phase or even by the addition of an organic carbon source.

In the algae according to the present embodiment, it is preferable that the growth rate in the logarithmic phase in the mixotrophic culture exceeds the growth rate in the logarithmic phase in the autotrophic culture and/or the growth rate in the logarithmic phase in the heterotrophic culture. This can be confirmed by measuring the weight (for example, dry weight) of the algal body per unit volume of the culture solution or measuring the absorbance of the culture medium (for example, absorbance ($OD_{750}$) at 750 nm). For example, in the algae according to the present embodiment, the specific growth rate calculated from the $OD_{750}$ on the 1st day and the 2nd day from the start of the culture is preferably 1 $\mu$/day or more and more preferably 1.2 $\mu$/day or more. More specifically, in the algae according to the present embodiment, the specific growth rate in a case of being cultured by mixotrophic culture under the culture conditions of Example 2 described later is preferably as described above. In a case of measuring the absorbance, it may be difficult to simply compare due to the types of algae, the culture density, and the like, and in such a case, it may be useful to use the weight.

The algae according to the present embodiment can proliferate under acidic conditions of pH 5 or less, and it is preferable that the algae can proliferate under acidic conditions of pH 4 or less, it is more preferable that the algae can proliferate under acidic conditions of pH 3 or less, it is still more preferable that the algae can proliferate under acidic conditions of pH 2 or less, and it is particularly preferable that the algae can proliferate under acidic conditions of pH 1 or less. It is preferable that the algae according to the present embodiment cannot proliferate under conditions of pH 7 or more. In acidic conditions of pH 1 or less, the proliferation of other organisms is suppressed. Therefore, the open culture can also be performed outdoors.

The algae according to the present embodiment are preferably algae belonging to the class Cyanidiophyceae. The algae belonging to the class Cyanidiophyceae grow well under conditions of a relatively high temperature of 30°C or higher and acidic conditions of pH 5 or less. The class Cyanidiophyceae is a freshwater unicellular alga classified into the class Cyanidiophyceae of the division Rhodophyta from a taxonomic viewpoint. Currently, four of the genera Cyanidioschyzon, Cyanidiococcus, Cyanidium, and Galdieria are classified in the class Cyanidiophyceae. The genus Cyanidiococcus is a genus newly reported as the class Cyanidiophyceae in recent years (Shao-Lun Liu et al., J. Phycol. 2020 Dec; 56(6): 1428-1442.). Cyanidium sp. YFU3 strain (FERM BP-22334) and Cyanidium sp. HKN1 strain (FERM BP-22333) reported in WO2019/107385A are classified into the genus Cyanidiococcus.

Examples of the algae belonging to the genus Cyanidioschyzon include Cyanidioschyzon merolae.

Examples of the algae belonging to the genus Cyanidiococcus include Cyanidiococcus yangmingshanensis.

Examples of the algae belonging to the genus Cyanidium include Cyanidium caldarium and Cyanidium sp.

Examples of the algae belonging to the genus Galdieria include Galdieria sulphuraria, Galdieria partita, Galdieria daedala, Galdieria maxima, and Galdieria phlegrea.

The algae belonging to the class Cyanidiophyceae may be isolated from an acidic environment such as an acidic hot spring, or may be obtained from a culture collection or the like. Examples of the culture collection include the Microbial Culture Collection at the National Institute for Environmental Studies (16-2 Onogawa, Tsukuba, Ibaraki, Japan), NITE Biological Resource Center (NBRC; 2-49-10 Nishihara, Shibuyaku, Tokyo, Japan), the Culture Collection of Algae at the GEORG-AUGUST-UNIVERSITY GOTTINGEN (SAG), and the American Type Culture Collection (ATCC; 10801 University Boulevard Manassas, VA 20110, USA).

The algae according to the present embodiment are preferably algae belonging to the genus Galdieria, the genus Cyanidium, or the genus Cyanidiococcus, and more preferably algae belonging to the genus Galdieria.

The algae according to the present embodiment may be haploids of algae belonging to the class Cyanidiophyceae or may be diploids, but is preferably haploids. The haploid of the algae belonging to the class Cyanidiophyceae often does not have a rigid cell wall as compared with the diploid cell. Therefore, the haploid cell of the algae belonging to the class Cyanidiophyceae can be lysed by relatively mild treatment such as neutralization treatment, hypotonic treatment, and freeze-thaw treatment. The algae according to the present embodiment may be algae belonging to the class Cyanidiophyceae that do not have rigid cell wall. In a case where the algae according to the present embodiment are diploids, the algae may be homozygous diploids of algae belonging to the class Cyanidiophyceae.

The algae according to the present embodiment can be produced from, for example, algae belonging to the class Cyanidiophyceae by the producion method described later.

The algae belonging to the class Cyanidiophyceae other than Cyanidioschyzon merolae are usually present as diploids in nature. Examples of a method for obtaining haploids from diploids of the algae belonging to the class Cyanidiophyceae include a method for culturing the diploid cells for a certain period of time. For example, the diploid cells are cultured in a liquid culture medium until the stationary phase, and the culture in the liquid culture medium is continued for any period of time while maintaining the stationary phase state, whereby the haploid cells can be obtained. Examples of the period for which the culture is continued in the stationary phase state include 2 to 60 days, 3 to 40 days, and 5 to 35 days. The period from the start of the culture to the stationary phase varies depending on the type of algae and the culture conditions. Alternatively, cells may be collected from the culture solution in the stationary phase, subcultured, and further cultured for about 1 to 5 days. By continuing the culture while maintaining the stationary phase state, some of the diploid cells undergo meiosis to produce haploid cells. As a result, the haploid cells are mixed in the diploid cells. The haploid cells of the algae belonging to the class Cyanidiophyceae usually have a smaller cell size than the diploid cells, and a rigid cell wall is not observed (WO2019/107385A). Therefore, the haploid cells and the diploid cells can be distinguished by optical microscope observation. The haploid cells appearing in the culture solution can be collected and isolated by a Pasteur pipette or the like to obtain the haploid cells. The obtained haploid cells can be maintained by subculturing using a solid culture medium or a liquid culture medium.

The determination of whether the algae are diploids or haploids can be performed by staining the cells with a nuclear staining reagent such as DAPI. For example, the cells to be determined and the cells known to be haploids are stained with a nuclear staining reagent. In a case where the cells to be determined exhibit the same fluorescence intensity as the cells known to be haploids, the cells to be determined can be determined to be haploids. In addition, in a case where the cells to be determined exhibit about twice the fluorescence intensity as the cells known to be haploids, the cells to be determined can be determined to be diploids. Alternatively, for example, the cells to be determined and the cells known to be diploids are stained with a nuclear staining reagent. In a case where the cells to be determined exhibit the same fluorescence intensity as the cells known to be diploids, the cells to be determined can be determined to be diploids. In addition, in a case where the cells to be determined exhibit about 1/2 times the fluorescence intensity as the cells known to be diploids, the cells to be determined can be determined to be haploids.

The determination of whether the algae are diploids or haploids may be performed by checking the copy number of the same genetic locus. For example, in a case where the copy number of the same genetic locus in the cells to be determined is 1, the cells to be determined can be determined to be haploids. It is also possible to determine that the algae are haploids by using a next-generation sequencer or the like. For example, whole genome sequence reads are acquired by a next-generation sequencer or the like, the sequence reads are assembled, and then the sequence reads are mapped for the sequence obtained by the assembling. In diploids, the difference in base between alleles are found in various regions on the genome. However, in haploids, because only one allele is present, no such regions are found. However, in this method, it is not possible to distinguish between the haploid cells and the homozygous diploid cells.

Since the haploids of the algae belonging to the class Cyanidiophyceae does not have a rigid cell wall, the cell wall is not usually observed in the observation (for example, 600 times magnification) by an optical microscope. Therefore, the cells to be determined may be determined to be haploids or diploids by observing the cells with an optical microscope or the like.

Since the haploid of the algae belonging to the class Cyanidiophyceae have no strong cell wall, it is possible to disrupt the cells by relatively mild treatment such as a neutralization treatment, a hypotonic treatment, or a freeze-thaw treatment. In the present specification, the description of "have no strong cell wall" means that cell rupture occurs by any of the following cell rupture treatments (A) to (C).

    (A) Algal cells are suspended in an isotonic solution at pH 7 and left for one week or longer.
    (B) Algal cells are suspended in distilled water and left for 1 minute or longer.
    (C) Algal cells are subjected to a drying treatment and suspended in an isotonic solution at pH 7.

In the above (A) to (C), in a case where the algal cells are cultured cells, the medium may be removed by centrifugation or the like before each treatment is performed, and the algal cells may be washed with an isotonic solution or the like.

In the above (A) and (C), examples of the isotonic solution include a pH 7 buffer containing 10% sucrose and 20 mM HEPES.

In the above (C), examples of the drying treatment include drying in a refrigerator (4°C), freeze-drying, and the like. For the drying treatment, the precipitate of algal cells collected by centrifugation is used. In a case where the cells are dried in a refrigerator, the drying treatment time depends on the amount of algal cells, and is, for example, 3 days or longer.

Whether or not the cells rupture has occurred can be determined by performing centrifugation (1,500x g, 3 minutes) on the algal cell suspension after the cell rupturing treatments (A) to (C) described above, and calculating a ratio of the mass of proteins in the centrifugal supernatant to the total mass of proteins in the algal cell suspension. Specifically, in a case where a rupture rate determined by the following expression is equal to or higher than 20%, it can be determined that cell rupture has occurred.

[Expression 1]

$$\text{Rupture rate} = \frac{\text{Protein mass (mass, g) of centrifuged supernatant}}{\text{Total protein mass (mass, g) of algal suspension}} \times 100 \ (\%)$$

Alternatively, in a case where the algal cells in the algal cell suspension are observed using an optical microscope (for example, at a magnification of 600X), and the ratio of ruptured cells to the total number of the algal cells is found to be about equal to or higher than 10% and preferably about equal to or higher than 20%, it may be determined that cell rupture has occurred.

In the cell rupturing treatments (A) to (C) described above, an isotonic solution at pH 7 can be used. Accordingly, it can be said that the cells ruptured by any of the cell rupturing treatments (A) to (C) are cells that are ruptured under the condition of pH 7.

Microalgae whose cells are ruptured under the condition of pH 7 are difficult to grow in an external environment in a case where the microalgae flow out of a culture tank, and contamination to the environment can be suppressed. Therefore, whether or not cell rupture occurs due to the mild hypotonic treatment under the condition of pH equal to or lower than 6 does not exert an influence on determining whether or not the microalgae have a rigid cell wall.

(Culture medium)

The algae according to the present embodiment can proliferate by assimilating sugar in a culture medium containing 0.1% by mass or more of sugar. The sugar is not particularly limited, and examples thereof include monosaccharides such as glucose, fructose, and galactose; disaccharides such as sucrose, lactose, and maltose; polysaccharides such as starch; and the like. It is preferable that the algae according to the present embodiment can proliferate by assimilating glucose in a culture medium containing 0.1% by mass or more of glucose.

The upper limit value of the concentration of the sugar contained in the culture medium is not particularly limited, and examples thereof include 20% by mass or less, preferably 10% by mass or less, and more preferably 5% by mass or less. Examples of the concentration range of the sugar contained in the culture medium include 0.1% to 20% by mass, preferably 0.5% to 10% by mass, and more preferably 1% to 5% by mass.

In a case where the sugar is glucose, the concentration of the glucose contained in the culture medium is preferably 1 mM or more, more preferably 10 mM or more, still more preferably 50 mM or more, and particularly preferably 100 mM or more. Examples of the concentration of the glucose contained in the culture medium include 1 to 1,000 mM, preferably 10 to 500 mM, more preferably 50 to 400 mM, still more preferably 100 to 300 mM, and particularly preferably 200 to 250 mM.

The pH of the culture medium is preferably pH 5 or less, more preferably pH 4 or less, still more preferably pH 3 or less, even more preferably pH 2 or less, and particularly preferably pH 1 or less.

The culture medium is not particularly limited, and an appropriate culture medium may be appropriately selected according to the type of algae to be cultured. Examples of the culture medium include a culture medium obtained by adding sugars to an inorganic salt culture medium containing a nitrogen source, a phosphorus source, trace elements (such as zinc, boron, cobalt, copper, manganese, molybdenum, and iron), and the like. For example, examples of the nitrogen source include ammonium salt, nitrate, nitrite, urea, amine, and the like, examples of the phosphorus source include phosphate and nitrite, and examples of the iron source include iron chloride, iron sulfate, iron citrate, and the like. Specific examples of the inorganic salt culture medium include a $2\times$ Allen medium (Allen MB. Arch. Microbiol. 1959 32: 270-277.), an M-Allen medium (Minoda A et al. Plant Cell Physiol. 2004 45: 667-71), an MA2 medium (Ohnuma M et al. Plant Cell Physiol. 2008 Jan; 49 (1): 11, 7-20.), and the like. In addition, in the present specification, the M-Allen medium is described as "MA medium" in some cases.

The culture medium may be a liquid culture medium or a solid culture medium, but is preferably a liquid culture medium. In a case of using a solid culture medium, a gelling agent such as gellan gum may be added to the liquid culture medium as described above to solidify the culture medium. Examples of the gelling agent include gellan gum, agarose, gelatin, and the like.

(Culture)

The algae according to the present embodiment have a feature that the chlorophyll content is 1 $\mu$g/mg dry weight or more even in a case of being cultured for 7 days under dark conditions in the presence of an organic carbon source (that is, under heterotrophic conditions). In a case of the solid culture medium, the culture may be performed by static culture. In a case of the liquid culture medium, it is preferable to supply oxygen appropriately by shaking culture or aeration culture.

It is preferable that the chlorophyll content of the algae according to the present embodiment is maintained at 1 $\mu$g/mg dry weight or more even in a case of being cultured for 7 days or more under heterotrophic conditions. It is preferable that the chlorophyll content of the algae according to the present embodiment is maintained semi-permanently even under heterotrophic conditions.

Since the chlorophyll content of the algae according to the present embodiment is maintained at 1 $\mu$g/mg dry weight or more even under heterotrophic conditions, in a case of performing mixotrophic culture, the algae can proliferate at a high density by utilizing both the assimilation of the organic carbon source and photosynthesis.

In the algae according to the present embodiment, for example, in a case of performing shaking culture (142 rpm) for 7 days at 42°C under dark conditions in a glucose-containing MA2 culture medium (200 mM glucose, pH 2.0), the chlorophyll content of the algal cells collected after the culture is 1 $\mu$g/mg dry weight or more. In the algae according to the present embodiment, in a case of being cultured under the above-described conditions, the chlorophyll content of the algal cells collected after the culture is preferably 1.5 $\mu$g/mg dry weight or more, more preferably 2 $\mu$g/mg dry weight or more, still more preferably 3 $\mu$g/mg dry weight or more, and particularly preferably 5 $\mu$g/mg dry weight or more.

In the algae according to the present embodiment, in a case of being cultured under the above-described conditions, the carotenoid content of the algal cells collected after the culture is preferably 0.7 $\mu$g/mg dry weight or more, more preferably 0.8 $\mu$g/mg dry weight or more, still more preferably 1 $\mu$g/mg dry weight or more, and particularly preferably 2 $\mu$g/mg dry weight or more.

It is preferable that in the algae according to the present embodiment, the vitamin C content of the algal cells obtained by mixotrophic culture is higher than the vitamin C content of the algal cells obtained by heterotrophic culture. For example, it is preferable that the vitamin C content in a case of being cultured by rotary shaking culture at 42°C, 120 $\mu$mol photons/m$^2$s, 2% $CO_2$, and 167 rpm using a $2\times$MA + 200 mM glucose (pH 1.0) culture medium is higher than the vitamin C content in a case of being cultured under dark conditions at the same conditions.

It is preferable that in the algae according to the present embodiment, the folate content of the algal cells obtained by mixotrophic culture is higher than the folate content of the algal cells obtained by heterotrophic culture. For example, it is preferable that the folate content in a case of being cultured by rotary shaking culture at 42°C, 120 $\mu$mol photons/m$^2$s, 2% $CO_2$, and 167 rpm using a $2\times$MA + 200 mM glucose (pH 1.0) culture medium is higher than the folate content in a case of being cultured under dark conditions at the same conditions.

It is preferable that in the algae according to the present embodiment, the dissolved oxygen concentration in the culture solution during mixotrophic culture is higher than the dissolved oxygen concentration in the culture solution during heterotrophic culture.

It is preferable that in the algae according to the present embodiment, the maximum cell density achieved in mixotrophic culture is higher than the maximum cell density achieved in heterotrophic culture.

[Method for high-density culture of unicellular algae]

A second aspect of the present invention relates to a method for high-density culture of unicellular algae, the method including mixotrophic culture of the unicellular algae. The unicellular algae are algae that can grow in a culture medium containing 0.1% by mass or more of sugars in acidic conditions of pH of 5 or less. The chlorophyll content of the unicellular algae after culture for 7 days under dark conditions in the presence of an organic carbon source is 1 $\mu$g/mg dry weight or more.

In the high-density culture method according to the present embodiment, the unicellular algae according to the first aspect can be used.

The mixotrophic culture may be performed by culturing in a light condition using a culture medium containing an organic carbon source.

Examples of the culture medium containing an organic carbon source include a culture medium obtained by adding an organic carbon source to the inorganic salt culture medium described above.

Examples of the organic carbon source include the above-described sugars; peptides such as peptone, tryptone, and casamino acid; sugar alcohols such as glycerol, sorbitol, and mannitol; and the like. The organic carbon source is preferably a sugar, preferably glucose or sucrose, and more preferably glucose.

Examples of the concentration of the organic carbon source in the culture medium include 10 to 1,000 mM. The concentration of the organic carbon source in the culture medium is preferably 100 mM or more, more preferably 150 mM or more, and still more preferably 200 mM or more. The upper limit value of the concentration of the organic carbon source in the culture medium is not particularly limited, and is, for example, 800 mM or less, 500 mM or less, 400 mM or less, or 300 mM or less.

The concentration of the organic carbon source contained in the culture medium is preferably 10 to 500 mM, more preferably 50 to 400 mM, still more preferably 100 to 300 mM, and particularly preferably 200 to 250 mM.

The pH of the culture medium is preferably acidic conditions of pH 5 or less, more preferably pH 4 or less, still more preferably pH 3 or less, even more preferably pH 2 or less, and particularly preferably pH 1 or less. By using the culture medium having a pH of 1 or less, the proliferation of other organisms can be suppressed. During the culture, it is preferable to maintain the pH of the culture solution at an acidic level, more preferably maintain the pH at 2 or less, and still more preferably maintain the pH at 1 or less. In a case where the pH of the culture solution is increased, the pH of the culture solution can be decreased by adding an acidic compound (sulfuric acid, hydrochloric acid, or the like) to the culture solution.

As a culture temperature, for example, 30°C to 50°C are exemplary examples, and 35°C to 50°C are preferable and 40°C to 50°C are more preferable. In a case of culturing the algae outdoors, it is preferable to culture the algae at a high temperature to prevent the proliferation of other organisms. The culture temperature in this case is preferably 35°C to 50°C.

Examples of the $CO_2$ condition include 0.04% to 5%, and the $CO_2$ condition is preferably 0.04% to 3%. The $CO_2$ condition may be an atmospheric $CO_2$ concentration. In a case where the algae are belonging to the genus Galdieria, the $CO_2$ concentration may be 100%.

The light condition may be a light intensity at which the algae can perform photosynthesis. Examples of the light intensity include 5 to 2,000 $\mu$mol photons/m$^2$s, and the light intensity is preferably 5 to 1,500 $\mu$mol photons/m$^2$s, more preferably 30 to 1,000 $\mu$mol photons/m$^2$s, and still more preferably 50 to 800 $\mu$mol photons/m$^2$s. In a case of culturing the algae outdoors, the algae may be cultured under sunlight. In a case where the algae are cultured indoors, the algae may be cultured under continuous light or go through a light-dark cycle (10L:14D or the like).

The culture may be static culture, aeration culture, or shaking culture. In a case of aeration culture, examples of the aeration conditions include 1 to 4 L air/min. In a case of shaking culture, examples of the shaking speed include 100 to 500 rpm, and the shaking speed is preferably 100 to 400 rpm.

The culture may be performed by batch culture, fed-batch culture, or continuous culture. The culture tank is not particularly limited, and for example, a fermenter, an outdoor culture tank, or the like can be used. The volume of the culture solution is not particularly limited, and can be appropriately selected according to the purpose. Examples of the volume of the culture solution include 100 mL to 1,000 L, 1 L to 500 L, 1 L to 300 L, and the like.

In the method according to the present embodiment, the algae can be proliferated at a high density by mixotrophic culture of the algae according to the first aspect. By mixotrophic culture, the algae can proliferate heterotrophically in the lower part or the deep part of the culture tank where the light is difficult to reach. On the other hand, the chlorophyll content of the algae according to the first aspect does not decrease even in the presence of an organic carbon source. Therefore, in the surface layer of the culture tank, the algae can proliferate autotrophically by photosynthesis. By maintaining the dissolved oxygen concentration in the culture solution high by photosynthesis, the heterotrophic proliferation is maintained high. By the heterotrophic proliferation and the autotrophic proliferation, the algae can proliferate at a high density even in a large-capacity culture tank.

By the proliferation of the algae at a high density, the value of the $OD_{750}$ of the culture solution is, for example, 30 or more, preferably 50 or more, more preferably 60 or more, and still more preferably 65 or more. The dry weight density of the algae in the culture solution depends on the type of the algae, but is usually limited to 0.4 g/L, but the class Cyanidiophyceae used in the present invention is 10 g/L or more. The dry weight density of the algae in the culture solution is preferably 20 to 30 g/L. In such a high-density culture state, it can be said that the sunlight does not reach the inside satisfactorily.

<Optional step>

The method according to the present embodiment may include an optional step in addition to the culture step. Examples of the optional step include a step of collecting the algae from the culture solution after the culture, a step of extracting a desired substance from the collected algal cells, and the like.

[Method for producing unicellular algae (1)]

A third aspect of the present invention is a method for producing unicellular algae, the method including: (ia) a step of producing haploid unicellular algae from diploid unicellular algae; (iia) a step of seeding the haploid unicellular algae on a solid culture medium containing an organic carbon source and culturing the seeded haploid unicellular algae under dark conditions; and (iiia) a step of collecting a green colony from colonies that have appeared on the solid culture medium.

(Step (ia))

In the step (ia), haploids of unicellular algae are produced from diploids of unicellular algae.
Examples of the diploids of unicellular algae include diploids of algae belonging to the class Cyanidiophyceae. The diploids of the algae belonging to the class Cyanidiophyceae is preferably diploids of the genus Cyanidiococcus, diploids of the genus Cyanidium, or diploids of the genus Galdieria, and more preferably diploid of the genus Galdieria.
The producion of the haploids of unicellular algae from the diploids of unicellular algae can be performed by the same method as the method described in the section of [Unicellular algae] above.

(Step (iia))

In the step (iia), the haploids of unicellular algae are seeded on a solid culture medium containing an organic carbon source and are cultured under dark conditions.
Examples of the organic carbon source include the same examples as those described above, and glucose is preferable.
Examples of the concentration of the organic carbon source in the culture medium include the same examples as those described above, and the concentration is preferably 100 to 300 mM and more preferably 200 to 250 mM.
Examples of the solid culture medium include a culture medium obtained by adding an organic carbon source and a gelling agent to the inorganic salt culture medium and solidifying the inorganic salt culture medium. Examples of the inorganic salt culture medium include the same examples as those described above.
The pH of the culture medium is preferably an acidic condition of pH 5 or less, more preferably pH 4 or less, and still more preferably pH 3 or less. The pH of the culture medium is preferably 0 to 2 and more preferably 1 to 2.
The culture may be static culture under dark conditions.
As a culture temperature, for example, 30°C to 50°C are exemplary examples, and 35°C to 50°C are preferable and 40°C to 50°C are more preferable.
Examples of the $CO_2$ condition include 0.04% to 5%, and the $CO_2$ condition is preferably 0.04% to 3%. The $CO_2$ condition may be an atmospheric $CO_2$ concentration.
The culture period is not particularly limited, and the culture may be performed until a colony appears on the solid culture medium. It is preferable to allow the colony to grow until it reaches a size at which the color can be discriminated after the appearance of the colony. For example, the culture may be performed until the colony diameter is about 1 to 10 mm.

(Step (iiia))

In the step (iiia), a green colony is collected from colonies that have appeared on the solid culture medium.
In the colonies that have appeared on the solid culture medium in the culture of the step (iia), there is a colony exhibiting a dark green color as compared with other colonies. The colony exhibiting the dark green color is collected from the solid culture medium with a platinum loop or the like.
The collected colony may be subcultured on another solid culture medium or may be subcultured on a liquid culture medium. The culture medium for subculture may be a culture medium containing an organic carbon source or a culture medium containing no organic carbon source, but is preferably a culture medium containing an organic carbon source.

The green colony can be selected, for example, as an indicator of the chlorophyll content. Algal cells are collected from the colony, and the chlorophyll content and the dry weight are measured by the method described in Examples later. From the measurement result, the chlorophyll content per unit dry weight is calculated, and a colony having a chlorophyll content of 1 $\mu$g/mg dry weight or more can be selected as the green colony.

Specific examples of the method for measuring the chlorophyll content include the following methods.

The collected colony is suspended in a culture medium to prepare an algal cell suspension, and the chlorophyll content and the dry weight are measured.

50 $\mu$L of the algal cell suspension is collected in a 1.5 mL tube, and centrifuged, and the supernatant is removed. 1 mL of dimethylformamide is added to the obtained pellet and dissolved. The absorbances of the dissolved solution at 647 nm and 664 nm are measured with an absorbance meter. From the obtained absorbance, the amount of chlorophyll per unit suspension ($\mu$g/mL) is calculated according to the method described in A. R. Wellburn (A. R. Wellburn, J. Plant Physiol. 144, 307-313 (1994)). This is corrected by the dry weight per unit suspension (mg/mL), and the chlorophyll content per dry weight ($\mu$g/mg) is calculated.

After measuring the weight (weight (A)) of the 2 mL tube, 1 mL of the algal cell suspension is collected in the 2 mL tube. The algal cell suspension put into the tube is centrifuged, and the supernatant is removed. The obtained pellet is frozen with liquid nitrogen and freeze-dried with a freeze dryer, and the weight (weight (B)) is measured. By subtracting the weight (A) from the weight (B), the dry weight per unit culture medium (mg/mL) is calculated.

In the method described in A. R. Wellburn, the concentration ($\mu$g/mL) of chlorophyll a (Ca), chlorophyll b (Cb), and carotenoid (Cx+c) per unit culture medium is calculated from the absorbance of the culture medium by the following equation.

$$Ca = 1.165A_{664} - 2.69A_{647}$$

$$Cb = 20.81A_{647} - 4.53A_{664}$$

$$Cx+c = (1000A_{480} - 0.89Ca - 52.02Cb)/245$$

$A_{664}$: absorbance of culture medium at 664 nm
$A_{647}$: absorbance of culture medium at 647 nm
$A_{480}$: absorbance of culture medium at 480 nm

The unicellular algae according to the first aspect can be produced by the productoin method according to the present embodiment. The unicellular algae obtained by the productoin method according to the present embodiment are haploids. The unicellular algae obtained by the productoin method according to the present embodiment are haploid unicellular algae having good growth in mixotrophic culture. Therefore, the method according to the present embodiment can also be said to be a method for producing haploid unicellular algae having good growth in mixotrophic culture.

[Method for producing unicellular algae (2)]

A third aspect of the present invention is a method for producing unicellular algae, the method including: (ib) a step of producing diploid unicellular algae from haploid unicellular algae; (iib) a step of seeding the diploid unicellular algae on a solid culture medium containing an organic carbon source and culturing the seeded diploid unicellular algae under dark conditions; and (iiib) a step of collecting a green colony from colonies that have appeared on the solid culture medium.

(Step (ib))

In the step (ib), diploids of unicellular algae are produced from haploids of unicellular algae.

Examples of the haploid unicellular algae include algae belonging to the class Cyanidiophyceae, and haploids of the genus Cyanidiococcus, haploids of the genus Cyanidium, or haploid of the genus Galdieria is preferable, and haploids of the genus Galdieria is more preferable.

In the producion of the diploid unicellular algae from the haploid unicellular algae, the diploid unicellular algae can be obtained by culturing the haploid unicellular algae in a liquid culture medium until the stationary phase, and continuing the culture in the liquid culture medium for any period of time while maintaining the stationary phase state. Examples of the period for which the culture is continued in the stationary phase state include 2 to 60 days, 3 to 40 days, and 5 to 35 days. The period from the start of the culture to the stationary phase varies depending on the type of algae and the culture conditions. Alternatively, cells may be collected from the culture solution in the stationary phase, subcultured, and further cultured for about 1 to 5 days. By continuing the culture in the stationary phase state, some of the haploid cells conjugate to produce diploid cells. As a result, the diploid cells are mixed in the haloid cells. The diploid cells of the algae belonging to the class Cyanidiophyceae usually have a larger cell size than the haploid cells thereof, and a rigid cell wall is observed (WO2019/107385A). Therefore, the haploid cells and the diploid cells can be distinguished by optical microscope observation. The diploid cells appearing in the culture solution can be collected and isolated by a Pasteur pipette or the like to obtain the diploid cells. The obtained diploid cells can be maintained by subculturing using a solid culture medium or a liquid culture medium.

The diploid cells obtained from one type of haploid cells are homozygous diploids. The homozygous diploid is a diploid in which two chromosome sets are completely the same as each other. The heterozygous diploid is a diploid in which two chromosome sets are different at least in part.

The determination of whether the diploid unicellular algae are homozygous diploids can be performed by checking the copy number of the same genetic locus. For example, in a case where the copy number of the same genetic locus in the cells to be determined is 1, the cells to be determined can be determined to be homozygous diploids. Alternatively, the determination of whether the diploid unicellular algas are homozygous diploids can also be performed using a next-generation sequencer or the like. For example, whole genome sequence reads are acquired by a next-generation sequencer or the like, the sequence reads are assembled, and then the sequence reads are mapped for the sequence obtained by the assembling. In the heterozygous diploid, the difference in base for each allele is found in various regions on the genome, but in the homozygous diploid, there is no difference in base for each allele, so such a region is not found.

(Step (iib))

In the step (iib), the diploids of unicellular algae are seeded on a solid culture medium containing an organic carbon source and are cultured under dark conditions.

The step (iib) can be performed by the same method as the step (iia).

The culture period is not particularly limited, and the culture may be performed until a colony appears on the solid culture medium. It is preferable to allow the colony to grow until it reaches a size at which the color can be discriminated after the appearance of the colony. For example, the culture may be performed until the colony diameter is about 1 to 10 mm.

(Step (iiib))

In the step (iiib), a green colony is collected from colonies that have appeared on the solid culture medium.

In the colonies that have appeared on the solid culture medium in the culture of the step (iib), there is a colony exhibiting a dark green color as compared with other colonies. The colony exhibiting the dark green color is collected from the solid culture medium with a platinum loop or the like.

The collected colony may be subcultured on another solid culture medium or may be subcultured on a liquid culture medium. The culture medium for subculture may be a culture medium containing an organic carbon source or a culture medium containing no organic carbon source, but is preferably a culture medium containing an organic carbon source. The green colony can be selected, for example, as an indicator of the chlorophyll content. For example, a colony having a chlorophyll content of 1 $\mu$g/mg dry weight or more can be selected as the green colony.

The algae according to the first aspect can be produced by the productoin method according to the present embodiment. The unicellular algae obtained by the productoin method according to the present embodiment are diploids. The algae obtained by the productoin method according to the present embodiment are diploid algae having good growth in mixotrophic culture. Therefore, the method according to the present embodiment can also be said to be a method for producing diploid algae having good growth in mixotrophic culture.

Examples

Hereinafter, the present invention will be described with reference to Examples, but the present invention is not limited to Examples.

(Algae)

Galdieria partita NBRC 102759 (obtained from Biological Resource Center, NITE (NBRC)) was used. The algal strain obtained from NBRC is a diploid.

(Culture medium)

The composition of the MA2 culture medium is shown in Table 1.

[Table 1]

| MA2 culture medium | |
| --- | --- |
| $(NH_4)_2SO_4$ | 5.28 g |
| $KH_2PO_4$ | 1.09 g |
| $MgSO_4 \cdot 7H_2O$ | 0.99 g |
| $CaCl_2 \cdot 2H_2O$ | 0.14 g |
| A2 trace element | 4 mL |
| Distilled water | 992 mL |
| Prepared with sulfuric acid to pH 1.0 or 2.0 | |
| 4 mL of A2 Fe stock is added after autoclave | |

| A2 trace element | |
| --- | --- |
| $H_3BO_4$ | 2.85 g |
| $MnCl_2 \cdot 4H_2O$ | 1.8 g |
| $ZnCl_2$ | 0.105 g |
| $Na_2MoO_4 \cdot 2H_2O$ | 0.39 g |
| $CoCl_2 \cdot 6H_2O$ | 40 mg |
| $CuCl_2 \cdot 2H_2O$ | 43 mg |
| Distilled water | 1,000 mL |

| A2 Fe stock | |
| --- | --- |
| EDTA-2Na | 7 g |
| $FeCl_3 \cdot 6H_2O$ | 6.8 g |
| Distilled water | 1,000 mL |
| Filter sterilization | |

The glucose-containing MA2 culture medium was prepared by adding 200 mM glucose to the MA2 culture medium. The pH of the culture medium was adjusted to 1.0 or 2.0.

The glucose-containing gellan gum plate was prepared by adding 0.5% (w/v) gellan gum (FUJIFILM Wako Pure Chemical Corporation) and 100 mM glucose to the MA2 culture medium. The pH of the culture medium was adjusted to 1.0 or 2.0.

The composition of the MA culture medium is shown in Table 2.

[Table 2]

| MA culture medium | |
| --- | --- |
| $(NH_4)_2SO_4$ | 2.64 g |
| $KH_2PO_4$ | 0.54 g |
| $MgSO_4 \cdot 7H_2O$ | 0.5 g |
| $CaCl_2 \cdot 2H_2O$ | 0.14 g |
| A2 trace element | 2 mL |
| Distilled water | 994 mL |
| Prepared with sulfuric acid to pH 1.0 or 2.0 | |
| 4 mL of A2 Fe stock is added after autoclave | |

| A2 trace element | |
| --- | --- |
| $H_3BO_4$ | 2.85 g |
| $MnCl_2 \cdot 4H_2O$ | 1.8 g |

(continued)

| A2 trace element | |
|---|---|
| $ZnCl_2$ | 0.105 g |
| $Na_2MoO_4 \cdot 2H_2O$ | 0.39 g |
| $CoCl_2 \cdot 6H_2O$ | 40 mg |
| $CuCl_2 \cdot 2H_2O$ | 43 mg |
| Distilled water | 1,000 mL |

| A2 Fe stock | |
|---|---|
| EDTA·2Na | 7 g |
| $FeCl_3 \cdot 6H_2O$ | 6.8 g |
| Distilled water | 1,000 mL |
| Filter sterilization | |

The composition of the 2×MA culture medium is shown in Table 3.

[Table 3]

| 2×MA culture medium | |
|---|---|
| $(NH_4)_2SO_4$ | 5.28 g |
| $KH_2PO_4$ | 1.09 g |
| $MgSO_4 \cdot 7H_2O$ | 0.99 g |
| $CaCl_2 \cdot 2H_2O$ | 0.28 g |
| A2 trace element | 2 mL |
| Distilled water | 990 mL |
| Prepared with sulfuric acid to pH 1.0 or 2.0 | |
| 8 mL of A2 Fe stock is added after autoclave | |

| A2 trace element | |
|---|---|
| $H_3BO_4$ | 2.85 g |
| $MnCl_2 \cdot 4H_2O$ | 1.8 g |
| $ZnCl_2$ | 0.105 g |
| $Na_2MoO_4 \cdot 2H_2O$ | 0.39 g |
| $CoCl_2 \cdot 6H_2O$ | 40 mg |
| $CuCl_2 \cdot 2H_2O$ | 43 mg |
| Distilled water | 1,000 mL |

| A2 Fe stock | |
|---|---|
| EDTA·2Na | 7 g |
| $FeCl_3 \cdot 6H_2O$ | 6.8 g |
| Distilled water | 1,000 mL |
| Filter sterilization | |

The seawater culture medium was obtained by replacing distilled water with seawater in the composition of the MA2 culture medium shown in Table 1. The glucose-containing seawater culture medium was prepared by adding 250 mM glucose to the seawater culture medium. The pH of the culture medium was adjusted to 1.0 or 2.0.

(Measurement of dry weight)

After measuring the weight (weight (A)) of the 2 mL tube, 1 mL of the culture solution was collected in the 2 mL tube. The culture solution put into the tube was centrifuged, and the supernatant was removed. The pellet was frozen with liquid nitrogen and freeze-dried with a freeze dryer (EYELA FDU-1200), and the weight (weight (B)) was measured. By subtracting the weight (A) from the weight (B), the dry weight per unit culture medium (mg/mL) was calculated. The dry weight was used to correct the chlorophyll content, the carotenoid content, and the protein content described later.

(Measurement of chlorophyll content and carotenoid content)

50 μL of the sample was collected in a 1.5 mL tube, and centrifuged, and the supernatant was removed. Thereafter, 1 mL of dimethylformamide was added to the pellet and the pellet was dissolved. The absorbances of the dissolved solution adjusted as described above at 480 nm, 647 nm, and 664 nm were measured with an absorbance meter (Biospectrometer basic; Eppendorf, Germany). From the obtained absorbance, the amounts of chlorophyll (μg/mL) and carotenoid (μg/mL) per unit culture medium were calculated according to the method described in A. R. Wellburn (A. R. Wellburn, J. Plant Physiol. 144, 307-313 (1994)). The calculated amounts of chlorophyll (μg/mL) and carotenoid (μg/mL) were corrected by the dry weight (mg/mL) per unit culture medium, and the amounts of chlorophyll (μg/mg) and carotenoid (μg/mg) per dry weight were calculated.

(Measurement of protein mass)

50 μL of the sample was collected in a 1.5 mL tube, and centrifuged, and the supernatant was removed. Thereafter, the pellet was frozen with liquid nitrogen and stored at -80°C. The pellet was suspended in a sample buffer (2% (w/v) SDS, 62 mM Tris-HCl, pH 6.8, 100 mM DTT, 10% (w/v) glycerol, 0.01% (w/v) bromophenol blue), and the suspension was adjusted to 100 μL. The quantification reagent was XL-Bradford (Integrale, Japan), and the protein mass (μg/mL) was quantified using bovine serum albumin (BSA) as a standard reagent. The calculated protein mass (μg/mL) was corrected by the dry weight (mg/mL) per unit culture medium, and the protein mass (μg/mg) per dry weight was calculated.

(Metabolome analysis)

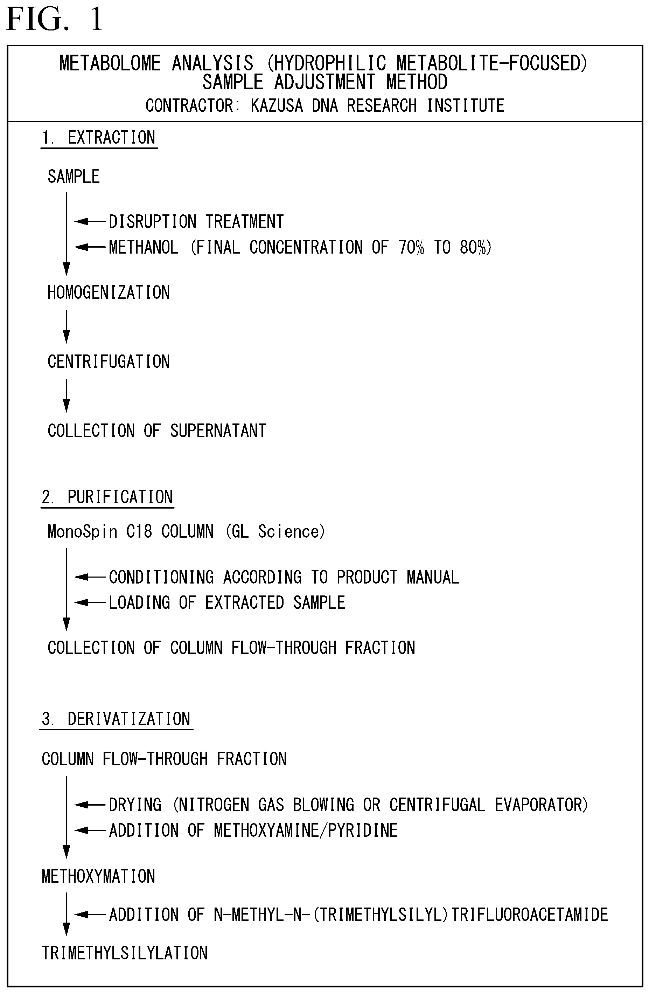

The metabolome analysis (hydrophilic metabolite focus analysis) was performed by KAZUSA DNA Research Institute. FIG. 1 shows a sample adjustment method in the metabolome analysis. The adjusted sample was analyzed by GC-MS. In the GC-MS analysis, an analysis was performed by a multiple reaction monitoring (MRM) method using a gas chromatograph-triple quadrupole mass spectrometer manufactured by Shimadzu Corporation. Table 4 shows main set values of the device used for the GS-MS analysis.

[Table 4]

| GC-MS | SIMADZU TQ-8050NX |
|---|---|
| Auto Sampler | SIMADZU AOC-30i |
| Column | RESTEK Rxi-5sil MS Integra-Guard (length of 30 m, inner diameter of 0.250 mm, film thickness of 0.50 μm) |
| Carrier gas | He, Constant flow |
| Vaporizer temperature | 250°C |
| Oven temperature | 80°C (2 min)-<br>12°C/min-320°C-<br>320°C (10 min) |
| Ionization method | EI |

(Measurement of growth status)

The growth status of the algae was confirmed by cell turbidity ($OD_{750}$). Specifically, the absorbance of the algae culture solution at 750 nm was measured using an absorbance meter (SmartSpec Plus of BIO-RAD) to obtain cell turbidity ($OD_{750}$). The algal culture solution was diluted 50 times in the culture medium and measured using the absorbance of the culture medium (pH 1.0, 100 mM glucose) at 750 nm as a blank, which was measured with an absorbance meter (Biospectrometer basic; Eppendorf, Germany).

(Measurement of dissolved oxygen concentration)

The dissolved oxygen concentration in the culture solution was measured with a dissolved oxygen meter (InPro 6000 Amperometric O2 Sensors; METTLER TOLEDO, Switzerland) that had been subjected to two-point calibration. The calibration of the dissolved oxygen meter was performed as follows. The measurement value of the dissolved oxygen meter was stabilized at room temperature in the air, and this measurement value was set to 100%. Next, the measurement value was stabilized by consuming oxygen in the reaction solution (sodium sulfite solution) by a chemical reaction method, and this measurement value was set to 0%.

[Example 1]

(Creation of haploid cells)

Galdieria partita NBRC 102759 (diploid) (hereinafter, also referred to as "2N strain") was cultured in the MA culture medium until it reached a plateau, the cell group was taken out from this culture solution, diluted 100 times, and further subcultured on the MA culture medium (pH 1.0). After 3 days of culturing, tadpole-like cells appeared. Therefore, by using a Pasteur pipette with a sharp tip, the tadpole-like cells were isolated, as haploid cells, from the culture solution while being observed under a microscope. The isolated haploid cells were cultured in the MA culture medium at 42°C, under illumination of 50 $\mu$mol photons/m$^2$s, in 2% $CO_2$.

(Creation of high-density growth strain)

The haploid cells produced as described above were seeded on a glucose-containing gellan gum plate (100 mM glucose, pH 2.0) and statically cultured at 42°C under dark conditions for about 2 weeks. FIG. 1 shows the colonies that have appeared after the static culture. For reference, the colonies of the 2N strain were also shown.

Among the colonies shown in FIG. 1, the dark green colonies (N1, N5, and N6 in FIG. 1) were collected as high-density growth strains.

(Comparison of photosynthetic pigment content in heterotrophic culture)

The colonies of N1 (hereinafter, also referred to as "N1 strain") and the colonies of N5 (hereinafter, also referred to as "N5 strain") shown in FIG. 1 were seeded on 50 mL of a glucose-containing 2×MA culture medium (200 mM glucose, pH 1.0) in a 100 mL Erlenmeyer flask, and cultured by shaking culture (142 rpm) for 7 days at 42°C under dark conditions.

As a comparative example, the colonies of N2 (hereinafter, also referred to as "N2 strain"), the colonies of N3 (hereinafter, also referred to as "N3 strain"), and the colonies of N4 (hereinafter, also referred to as "N4 strain") shown in FIG. 1 were seeded on 50 mL of a glucose-containing 2×MA culture medium (200 mM glucose, pH 1.0) in a 100 mL Erlenmeyer flask, and cultured by shaking culture (142 rpm) for 7 days at 42°C under dark conditions.

The algal cells were collected from each culture solution after the culture, and the dry weight per unit culture medium was measured. In addition, the chlorophyll and carotenoid were measured, and the content per dry weight was calculated. The results thereof are listed in Table 5. It was confirmed that the N1 strain and the N5 strain had higher chlorophyll content and carotenoid content than the N2 to N4 strains.

[Table 5]

| Algal strain | Maximum $OD_{750}$ achieved | Chlorophyll ($\mu$g/mg dry weight) | Carotenoid ($\mu$g/mg dry weight) |
|---|---|---|---|
| N1 | 12.70 | 6.42 | 2.44 |
| N5 | 20.15 | 1.95 | 0.88 |
| N2 | 19.30 | 0.74 | 0.47 |
| N3 | 28.30 | 0.72 | 0.60 |
| N4 | 28.02 | 0.69 | 0.45 |

The N1 strain and the N2 strain were seeded on 1 L of a glucose-containing 2×MA culture medium (200 mM glucose, pH 1.0) in a 2 L Erlenmeyer flask, and cultured by shaking culture (142 rpm) for 9 days (N1 strain) or 8 days (N2 strain) at 42°C under dark conditions. The algal cells were collected from the culture solution after the culture, and the dry weight per unit culture medium was measured. In addition, the chlorophyll, carotenoid, and protein were measured, and the contents per dry weight thereof were calculated. The results are listed in Table 6.

[Table 6]

| Algal strain | MaximumOD$_{750}$ achieved | Chlorophyll ($\mu$g/mg dry weight) | Carotenoid ($\mu$g/mg dry weight) | Protein ($\mu$g/mg dry weight) |
|---|---|---|---|---|
| N1 | 10.56 | 1.52 | 1.12 | 44.38 |
| N2 | 33 | 0 | 0.33 | 31.2 |

[Example 2]

(Comparison of specific growth rate in mixotrophic culture and heterotrophic culture)

The mixotrophic culture and the heterotrophic culture of the N1 strain and the N2 strain were performed under the following conditions. The OD$_{750}$ on the 1st day and the 2nd day of the culture was measured, and the specific growth rates of the N1 strain and the N2 strain in each culture were calculated.

<Culture conditions>

Culture medium used: 20 mL of 2×MA + 200 mM glucose (pH 1.0)
Culture container: 25 cm$^2$ cell culture flask (90026, TPP Techno Plastic Products)
Illumination condition: 50 $\mu$mol photons/m$^2$s (mixotrophic culture) or dark (heterotrophic culture)
Temperature condition: 40°C
CO$_2$ condition: 2% CO$_2$ inside incubator
Shaking speed: static culture
Culture start OD$_{750}$: 0.2

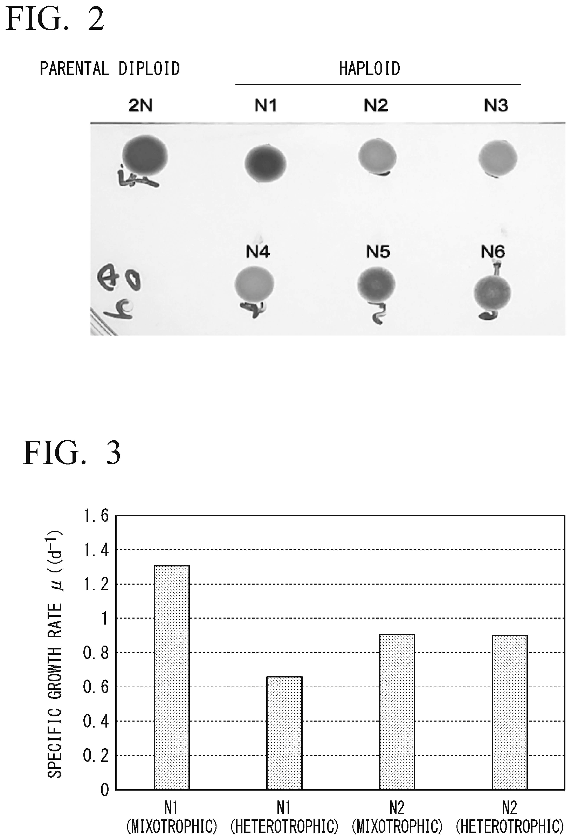

The results are shown in FIG. 3. In the N1 strain, the specific growth rate in the mixotrophic culture was significantly improved as compared with the heterotrophic culture. On the other hand, in the N2 strain, there was no difference in the specific growth rate between the mixotrophic culture and the heterotrophic culture. The specific growth rate of the N1 strain in the mixotrophic culture was higher than the specific growth rate of the N2 strain in the mixotrophic culture or the heterotrophic culture.
From these results, it was confirmed that the N1 strain exhibited excellent growth in the mixotrophic culture.

[Example 3]

(Comparison of vitamin C content in mixotrophic culture and heterotrophic culture)

The mixotrophic culture and the heterotrophic culture of the N1 strain and the N2 strain were performed under the following conditions. After the culture, the algal cells were collected from each culture solution, and the metabolome analysis was performed. The algal cells were collected on the 4th day of the culture in the N1 strain, and the algal cells were collected on the 3rd day of the culture in the N2 strain.

<Culture conditions>

Culture medium used: 2×MA + 200 mM glucose (pH 1.0)
Culture container: baffled Erlenmeyer flask (300 mL)
Illumination condition: 120 $\mu$mol photons/m$^2$s (mixotrophic culture) or dark (heterotrophic culture)
Temperature condition: 42°C
CO$_2$ condition: 2% CO$_2$ inside incubator
Shaking speed: 167 rpm (rotary shaking)
Culture start OD$_{750}$: 0.5

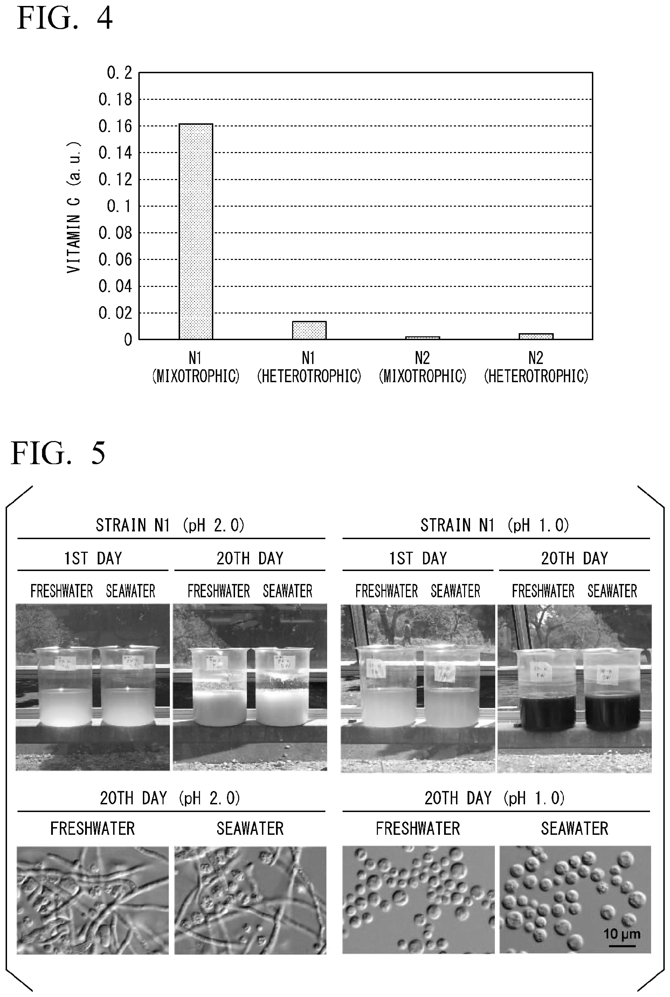

FIG. 4 shows the results of analyzing the vitamin C content of the algal cells by the metabolome analysis. In the N1 strain, the vitamin C content was higher than that of the N2 strain in both the mixotrophic culture and the heterotrophic culture. In the N1 strain, the vitamin C content in the mixotrophic culture was significantly improved as compared with the heterotrophic culture. On the other hand, in the N2 strain, there was no difference in the vitamin C content between the mixotrophic culture and the heterotrophic culture.

From these results, it was confirmed that the N1 strain had a significantly improved vitamin C content in the mixotrophic culture.

[Example 4]

(Metabolome analysis)

Tables 7 to 11 show the results of the metabolome analysis performed in Example 3. In Tables 7 to 11, each group is as follows. The algal cells having the dry weight described in each group were weighed, dissolved in a solvent to a certain concentration, and a part of the solution was used for the metabolome analysis.

Group B: mixotrophic culture strain of N1 (GpN1_M) 5.02 mg
Group C: heterotrophic culture strain of N1 (GpN1_H) 5.07 mg
Group D: mixotrophic culture strain of N2 (GpN2_M) 5.02 mg
Group E: heterotrophic culture strain of N2 (GpN2_H) 5.06 mg

[Table 7]

| Group | B | C | D | E |
|---|---|---|---|---|
|  | GpN1_M | GpN1_H | GpN2_M | GpN2_H |
| alkane C10 | 0.146758 | 0.144379 | 0.148032 | 0.14701 |
| Pyruvic acid | 0.002996 | 0.001589 | 0.003947 | 0.003858 |
| Lactic acid | 1.653564 | 0.442948 | 2.080748 | 1.719258 |
| Glycolic acid | 0.011936 | 0.011299 | 0.011023 | 0.013194 |
| Alanine | 4.23516 | 2.344118 | 7.905587 | 6.901546 |
| Alanine 13C3 |  |  |  |  |
| alkane C11 |  | 0.123973 | 0.137469 | 0.137018 |
| 2-Hydroxybutyric acid | 0.000436 | 0.000352 | 0.000475 | 0.000494 |
| Oxalic acid | 0.002421 | 0.003488 | 0.001869 | 0.001606 |
| 2-Aminoisobutyric acid |  |  |  |  |
| 3-Hydroxybutyric acid | 0.001932 | 0.001585 | 0.001649 | 0.00198 |
| 2-Hydroxyisovaleric acid |  |  |  |  |
| 2-Aminobutyric acid | 0.002911 | 0.000968 | 0.014804 | 0.015554 |
| Malonic acid | 0.000276 | 0.000351 | 0.000196 | 0.000208 |
| alkane C12 | 0.141847 | 0.143698 | 0.141004 | 0.143042 |
| Valine | 1.148984 | 0.707103 | 1.271396 | 1.318958 |
| Valine 13C5,15N |  |  |  |  |
| Urea |  |  |  |  |
| Benzoic acid | 0.079618 | 0.065538 | 0.075874 | 0.076396 |
| Glycerol | 1.592289 | 0.757822 | 1.645124 | 1.716878 |
| Phosphoric acid | 1.087825 | 1.085596 | 1.073334 | 1.060707 |
| Leucine d3 |  |  |  |  |
| Leucine | 0.159671 | 0.120321 | 0.213939 | 0.260644 |
| Isoleucine | 0.165723 | 0.139611 | 0.188778 | 0.197443 |

(continued)

| Group | B | C | D | E |
|---|---|---|---|---|
| | GpN1_M | GpN1_H | GpN2_M | GpN2_H |
| Isoleucine 13C6,15N | | | | |
| Maleic acid | 0.006615 | 0.005273 | 0.009509 | 0.009204 |
| Proline | 19.228762 | 15.031253 | 24.641393 | 25.500818 |
| Proline 13C5,15N | | | | |
| Nicotinic acid | 0.001678 | 0.000898 | 0.001486 | 0.001712 |
| alkane C13 | 0.135969 | 0.139347 | 0.141122 | 0.136862 |
| Glycine | 0.518993 | 0.238873 | 0.777074 | 0.649928 |
| Glycine 13C2,15N | | | | |
| Succinic acid | 0.012346 | 0.006896 | 0.015656 | 0.013883 |
| Picolinic acid | | | | |
| Uracil | 0.029333 | 0.017814 | 0.041242 | 0.043848 |
| Fumaric acid | 0.040568 | 0.019327 | 0.055711 | 0.043957 |
| Serine | 6.901887 | 3.932819 | 5.965762 | 6.381726 |
| Serine 13C3,15N | | | | |
| Threonine | 0.668529 | 0.2981 | 0.864516 | 0.804293 |
| Threonine 13C4 | | | | |
| Thymine | 0.001454 | 0.001355 | 0.001665 | 0.001837 |
| Glutaric acid | 0.000703 | 0.000878 | 0.00061 | 0.000576 |
| alkane C14 | 0.704746 | 0.711165 | 0.707682 | 0.700322 |
| 3-Aminopropanoic acid | 0.088934 | 0.063126 | 0.039747 | 0.063396 |
| Fatty acid C10:0 | 0.001539 | 0.001368 | 0.001316 | 0.001495 |

[Table 8]

| Group | B | C | D | E |
|---|---|---|---|---|
| | GpN1_M | GpN1_H | GpN2_M | GpN2_H |
| alkane C14 | 0.704746 | 0.711165 | 0.707682 | 0.700322 |
| 3-Aminopropanoic acid | 0.088934 | 0.063126 | 0.039747 | 0.063396 |
| Fatty acid C10:0 | 0.001539 | 0.001368 | 0.001316 | 0.001495 |
| 3-Aminoisobutyric acid | 0.00187 | 0.00166 | 0.000523 | 0.00072 |
| Malic acid | 0.106176 | 0.045944 | 0.139832 | 0.108274 |
| Nicotinamide | 0.066754 | 0.026502 | 0.070054 | 0.07436 |
| Erythritol | 0.001524 | 0.000844 | 0.001297 | 0.001232 |
| Adipic acid | 0.000801 | 0.000799 | 0.000527 | 0.000442 |
| alkane C15 | 1.688175 | 1.686125 | 1.676397 | 1.665643 |
| Aspartic acid d3 | | | | |
| Aspartic acid | 1.383773 | 1.043174 | 1.492276 | 1.277137 |
| Methionine | 0.062469 | 0.038116 | 0.067795 | 0.087788 |
| Methionine 13C5,15N | | | | |

(continued)

| Group | B | C | D | E |
|---|---|---|---|---|
| | GpN1_M | GpN1_H | GpN2_M | GpN2_H |
| Cytosine | 0.005261 | 0.006139 | 0.00449 | 0.005031 |
| Pyroglutamic acid | 0.371817 | 0.274425 | 0.447782 | 0.543721 |
| 4-Aminobutyric acid | 0.179253 | 0.131277 | 0.372272 | 0.371907 |
| Cysteine | 0.007776 | 0.003224 | 0.011892 | 0.012084 |
| Creatinine | | | | |
| 2-Hydroxyglutaric acid | 0.001406 | 0.001027 | 0.001599 | 0.001108 |
| 2-Ketoglutaric acid | 0.000479 | 0.000293 | 0.000729 | 0.000744 |
| 2-Isopropylmalic acid | | | | |
| Hypotaurine | | | | |
| alkane C16 | | | | |
| Glutamic acid | 3.625063 | 2.371717 | 4.41134 | 5.1702 |
| Glutamic acid 13C5,15N | | | | |
| Anthranilic acid | | | | |
| Tartaric acid | | | | |
| Phenylalanine | 0.149931 | 0.103347 | 0.184855 | 0.185228 |
| Phenylalanine 13C9,15N | | | | |
| Xylose | 0.000437 | 0.001021 | 0.000591 | 0.000862 |
| Arabinose | 0.000874 | 0.000568 | 0.000763 | 0.000763 |
| Fatty acid C12:0 | 0.002392 | 0.004059 | 0.002093 | 0.003135 |
| Asparagine | 2.704124 | 1.458337 | 2.073568 | 2.169054 |
| Asparagine 13C4,15N2 | | | | |
| Ribose | 0.007547 | 0.005005 | 0.009428 | 0.010481 |
| Taurine | 0.913035 | 0.599589 | 0.826234 | 0.75164 |
| Taurine 13C2 | | | | |
| Xylitol | 0.001993 | 0.001043 | 0.002745 | 0.00227 |
| 2-Aminoadipic acid | 0.001205 | 0.001028 | 0.002281 | 0.001822 |
| alkane C17 | 1.062744 | 1.075846 | 1.07614 | 1.06323 |
| Arabitol | 0.010958 | 0.005309 | 0.012051 | 0.010946 |
| Ribitol | 0.062984 | 0.026942 | 0.075649 | 0.060987 |
| Ribitol 13C5 | | | | |
| Indoxyl sulfate | | | | |
| Quinolinic acid | | | | |

[Table 9]

| Group | B | C | D | E |
|---|---|---|---|---|
| | GpN1_M | GpN1_H | GpN2_M | GpN2_H |
| Dihydroxyacetone phosphate | 0.001509 | 0.000937 | 0.001536 | 0.001649 |
| Aconitic acid | 0.002277 | 0.002244 | 0.004068 | 0.005548 |

(continued)

| Group | B | C | D | E |
|---|---|---|---|---|
| | GpN1_M | GpN1_H | GpN2_M | GpN2_H |
| Orotic acid | | | | |
| Putrescine | 0.010585 | 0.01018 | 0.017472 | 0.014559 |
| 3-Hydroxyanthranilic acid | | | | |
| Glutamine | 5.907959 | 3.036883 | 5.141158 | |
| Glutamine 13C5,15N2 | | | | |
| Homovanillic acid | | | | |
| Dihydroorotic acid | | | | |
| Shikimic acid | | | | |
| 3-Phosphoglyceric acid | 0.001333 | 0.000784 | 0.000944 | 0.00109 |
| Isocitric acid | 0.00055 | 0.000253 | 0.001048 | 0.001144 |
| alkane C18 | 1.148431 | 1.148354 | 1.128344 | 1.126268 |
| Citric acid | 0.031574 | 0.040384 | 0.063204 | 0.116668 |
| Hypoxanthine | 0.026314 | 0.016771 | 0.051609 | 0.056023 |
| Ornithine | 0.159089 | 0.108874 | 0.182787 | 0.182539 |
| Ornithine 13C5 | | | | |
| Arginine | 0.246007 | 0.149538 | 0.255776 | 0.317509 |
| Arginine 15N4 | | | | |
| 3,4-Dihydroxybenzeneacetic acid | | | | |
| Spermidine-3TMS | | | | |
| Fatty acid C14:0 | 0.001783 | 0.001548 | 0.001133 | 0.001175 |
| Indole-3-ethanol | | | | |
| Quinic acid | | | | |
| Fructose | 0.005284 | 0.002296 | 0.003921 | 0.00398 |
| Caffeine | | | | |
| Mannose | | | | |
| Gluconolactone | | | | |
| Adenine | 0.034901 | 0.050413 | 0.035462 | 0.032042 |
| Galactose | 0.558226 | 0.274409 | 0.332612 | 0.401272 |
| Histamine | | | | |
| Glucose | 1.585959 | 1.274278 | 0.605967 | 0.407018 |
| Erythrose 4-phosphate | | | | |
| Pyridgxine | | | | |
| alkane C19 | 1.205089 | 1.198932 | 1.182645 | 1.164295 |
| Indole-3-acetaldehyde | | | | |
| Histidine | 0.08665 | 0.036589 | 0.106937 | 0.095566 |
| Histidine 13C6,15N3 | | | | |
| Lysine | 0.128257 | 0.06158 | 0.126441 | 0.109428 |
| Lysine 13C6,15N2 | | | | |

(continued)

| Group | B | C | D | E |
|---|---|---|---|---|
| | GpN1_M | GpN1_H | GpN2_M | GpN2_H |
| Tyramine | | | | |
| Mannitol | 0.022404 | 0.011243 | 0.022258 | 0.019605 |
| Sorbitol | 0.033322 | 0.010959 | 0.020103 | 0.015191 |
| Galactitol | 0.083861 | 0.038147 | 0.034844 | 0.038217 |
| Tyrosine | 0.185587 | 0.084875 | 0.149275 | 0.124809 |

[Table 10]

| Group | B | C | D | E |
|---|---|---|---|---|
| | GpN1_M | GpN1_H | GpN2_M | GpN2_H |
| Tyrosine 13C6 | | | | |
| Ascorbic acid | 0.136123 | 0.013253 | 0.002583 | 0.004469 |
| Epinephrine | | | | |
| Indole-3-acetic acid | | | | |
| N-Acetylornithine | 0.006271 | 0.006989 | 0.003955 | 0.005965 |
| alkane C20 | 1.263372 | 1.263885 | 1.253727 | 1.246084 |
| 2-Deoxyribose 5-phosphate | | | | |
| Xanthine | 0.016374 | 0.006439 | 0.02738 | 0.02144 |
| Fatty acid C6:1 | | | | |
| Indole-3-carboxylic acid | | | | |
| Fatty acid C16:0 | 0.009012 | 0.018089 | 0.010421 | 0.006552 |
| 3-Methoxytyramine | | | | |
| Indole-3-propionic acid | | | | |
| Kynurenic acid | | | | |
| Dopamine | | | | |
| Ribulose 5-phosphate | | | | |
| Uric acid | | | | |
| Ribose 5-phosphate | 0.004467 | 0.00218 | 0.003845 | 0.003964 |
| Inositol | 0.386846 | 0.229876 | 0.268024 | 0.272096 |
| Dopa | | | | |
| Xylulose 5-phosphate | | | | |
| alkane C21 | 1.244616 | 1.251033 | 1.225758 | 1.232828 |
| Indole-3-acetamide | | | | |
| Guanine | 0.001604 | 0.008162 | 0.000775 | 0.000944 |
| Indole-3-lactic acid | | | | |
| Norepinephrine | | | | |
| Indole-3-butyric acid | | | | |
| Kynurenine | | | | |
| Indole-3-pyruvic acid | | | | |

(continued)

| Group | B | C | D | E |
|---|---|---|---|---|
| | GpN1_M | GpN1_H | GpN2_M | GpN2_H |
| alkane C22 | 1.254426 | 1.289086 | 1.24904 | 1.254154 |
| 5-Hydroxyindole-3-acetic acid | | | | |
| Fatty acid C18:2 | | | | |
| Fatty acid C18:1 | | | | |
| Fatty acid C18:3 | | | | |
| Tryptophan | 0.024109 | 0.013687 | 0.025449 | 0.027759 |
| Tryptophan 13C11,15N2 | | | | |
| Tryptamine | | | | |
| Fatty acid C18:0 | 0.006808 | 0.017863 | 0.010513 | 0.005135 |
| Xanthurenic acid | | | | |
| Cystine | 0.002747 | 0.001869 | 0.001615 | 0.001326 |
| Cystine d4 | | | | |
| Fructose 6-phosphate | 0.024075 | 0.010652 | 0.024358 | 0.023144 |
| alkane C23 | 1.343966 | 1.347375 1.347375 | 1.338439 | 1.337076 |
| Glucose 6-phosphate | 0.087359 | 0.038226 | 0.083348 | 0.077001 |
| Spermine-4TMS | | | | |

[Table 11]

| Group | B | C | D | E |
|---|---|---|---|---|
| | GpN1_M | GpN1_H | GpN2_M | GpN2_H |
| Fatty acid C20:4 | | | | |
| Fatty acid C20:5 | | | | |
| Melatonin- 2TMS | | | | |
| Iudole-3-acrylic acid | | | | |
| alkane C24 | 1.327547 | 1.321112 | 1.321801 | 1.330471 |
| 6-Phosphogluconic acid | | | | |
| Fatty acid C20:0 | | | | |
| Uridine | 0.000415 | 0.000178 | 0.001013 | 0.001227 |
| 5-Hydroxytryptophan | | | | |
| Melatonin-TMS | | | | |
| Serotonin-4TMS | | | | |
| Serotonin-3TMS | | | | |
| alkane C25 | 1.236333 | 1.222444 | 1.21347 | 1.241544 |
| Sedoheptulose 7-phosphate | | | | |
| Inosine | 0.00194 | 0.000967 | 0.006996 | 0.008168 |
| Fatty acid C22:6 | | | | |
| alkane C26 | 1.264616 | 1.265502 | 1.268445 | 1.282202 |
| Fatty acid C22:1 | | | | |

(continued)

| Group | B | C | D | E |
|---|---|---|---|---|
| | GpN1_M | GpN1_H | GpN2_M | GpN2_H |
| Sucrose | 0.018871 | 0.010644 | 0.051255 | 0.060012 |
| Adenosine | 0.004249 | 0.002614 | 0.003646 | 0.004299 |
| Fatty acid C22:0 | | | | |
| Lactose | | | | |
| Cytidine | 0.002609 | 0.001041 | 0.004053 | 0.00611 |
| alkane C27 | 1.278401 | 1.29117 | 1.276579 | 1.294292 |
| Maltose | 0.005524 | 0.003919 | 0.001031 | 0.001183 |
| Trehalose | | | | |
| Guanosine | 0.011277 | 0.004505 | 0.028087 | 0.033743 |
| alkane C28 | 1.332939 | 1.331952 | 1.304733 | 1.314583 |
| Fatty acid C24:1 | | | | |
| Uridine 5' -monophosphate | 0.022435 | 0.007889 | 0.024883 | 0.028648 |
| alkane C29 | 1.339744 | 1.337015 | 1.299587 | 1.310628 |
| alkane C30 | 2.737252 | 2.72714 | 2.648142 | 2.688423 |
| Adenosine 5' -monophosphate | 0.368509 | 0.114492 | 0.330462 | 0.35244 |
| alkane C31 | 2.69544 | 2.660475 | 2.573748 | 2.613012 |
| alkane C32 | 2.617382 | 2.605426 | 2.475706 | 2.518079 |
| Ergosterol | | | | |
| alkane C33 | 2.533515 | 2.52927 | 2.378713 | 2.425513 |
| Raffinose | | | | |

[Example 5]

(Open culture in mixotrophic culture)

The mixotrophic culture of N1 strain was performed under the following conditions. During the culture, sampling was performed over time, and the $OD_{750}$ was measured. On the 20th day of the culture, the culture solution was sampled and observed with an optical microscope. Since the water of the culture medium evaporated because of open culture, water was added during the culture period to maintain a constant amount of the culture medium.

<Culture conditions>

Culture medium used: 1 L of MA2 + 0.25 M glucose culture medium (pH 1.0 or pH 2.0) or 1 L of seawater + 0.25 M glucose culture medium (culture started at pH 1.0 or pH 2.0; pH adjustment with sulfuric acid)
Culture container: beaker (2 L)
Illumination condition, temperature condition, $CO_2$ condition: in a greenhouse (uncontrolled)

Room temperature: change in a range of 29.5°C to 44°C during the culture period was observed.
Culture medium temperature: change in a range of 29.3°C to 40.1°C during the culture period was observed.

Shaking speed: 140 rpm (rotary shaking)
Culture start $OD_{750}$: 1.0

FIG. 5 shows photographs of culture solutions on the 0th day and the 20th day and a microscopic photograph of a culture solution on the 20th day. In a case where the culture medium having a pH of 2.0 was used, the N1 strain died due to the proliferation of mold. On the other hand, in a case where the culture was started in the culture medium having a pH of 1.0, the proliferation of mold was not confirmed, and the proliferation of the N1 strain was confirmed.

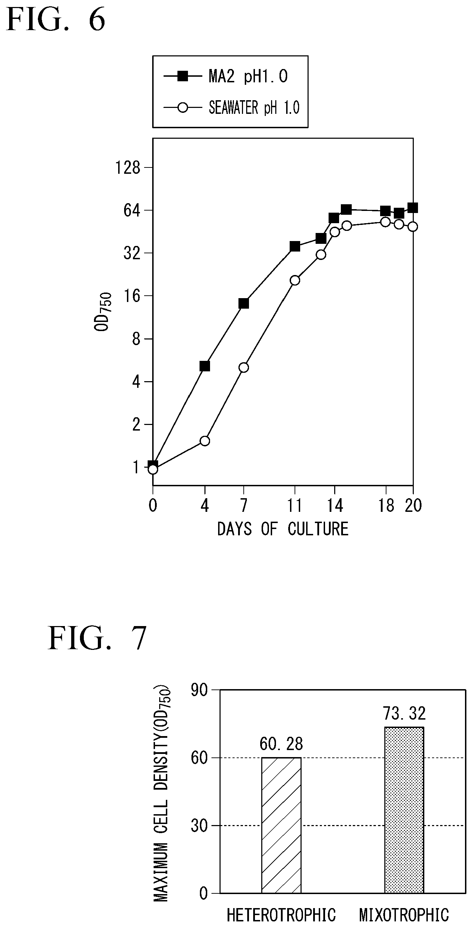

FIG. 6 shows the growth of the N1 strain in the culture medium having a pH of 1.0. There was no significant difference in the growth between the MA2 + 0.25 M glucose culture medium and the seawater + 0.25 M glucose culture medium.

The algal cells were collected from the culture solution on the 20th day of the culture, and the dry weight per unit culture medium was measured. In the MA2 + 0.25 M glucose culture medium, the dry weight was 13.72 g/L ($OD_{750}$ value: 65.6), and in the seawater + 0.25 M glucose culture medium, the dry weight was 11.72 g/L ($OD_{750}$ value: 49.0). The algae were filtered from the culture medium by centrifugation.

From these results, it was confirmed that, by culturing in a culture medium having a pH of 1.0, the algal strain could be proliferated while preventing the proliferation of mold or the like, even when open culture was performed in a culture medium containing an organic substance.

[Example 6]

(Pre-culture)

The N1 strain that had been cultured by heterotrophic culture in a Sakaguchi flask ($OD_{750}$ = 6.65, 200 ml, culture temperature of 40°C) was cultured by mixotrophic culture for 3 days in 350 mL of a culture medium ($2\times$MA + 0.2 M glucose, pH 2) using two 1-L cotton filters while being aerated (850 ml/min) (culture start $OD_{750}$ = 1, light intensity: 100 $\mu$mol photons/m$^2$s, culture temperature of 40°C).

After 3 days of culture, $OD_{750}$ = 11.5. Next, the mixotrophic culture was performed for 5 days in 5,000 ml of a culture medium ($2\times$MA + 0.2 M glucose, pH 2) using a fermenter (culture start $OD_{750}$ = 1.42, light intensity: 100 $\mu$mol photons/m$^2$s, culture temperature of 40°C). During the culture, the intensity of light was appropriately adjusted. On the 2nd day from the start of the culture in the fermenter, the light intensity of the fermenter that performs mixotrophic culture in the main culture was increased to 250 $\mu$mol photons/m$^2$s. On the 3rd day from the start of the culture in the fermenter, the light intensity of the fermenter that performs mixotrophic culture in the main culture was increased to the maximum (500 $\mu$mol photons/m$^2$s).

(Main culture)

5 days after the start of the culture in the fermenter, $OD_{750}$ = 62. The mixotrophic culture or the heterotrophic culture was performed for 3 days in 5,000 ml of a culture medium ($2\times$MA + 0.2 M glucose, pH 2) using a fermenter (culture start $OD_{750}$ = 8.7, light intensity of heterotrophic culture: 0 $\mu$mol photons/m$^2$s; light intensity of mixotrophic culture: 300 $\mu$mol photons/m$^2$s, culture temperature of 40°C). During the culture, the stirring speed of the fermenter was appropriately adjusted (culture start: 200 rpm, 2nd day of main culture: 300 rpm, 3rd day of main culture and later: 350 rpm). On the 2nd day of the main culture, the light intensity of the fermenter for mixotrophic culture was increased to the maximum (500 $\mu$mol photons/m$^2$s).

(Measurement of maximum cell density achieved)

After the main culture for 3 days, the $OD_{750}$ of the culture solution was measured. The results are shown in FIG. 7. From the results of FIG. 7, it was confirmed that, in the mixotrophic culture, the algal cells increased by 20% or more as compared with the heterotrophic culture.

(Measurement of dissolved oxygen concentration)

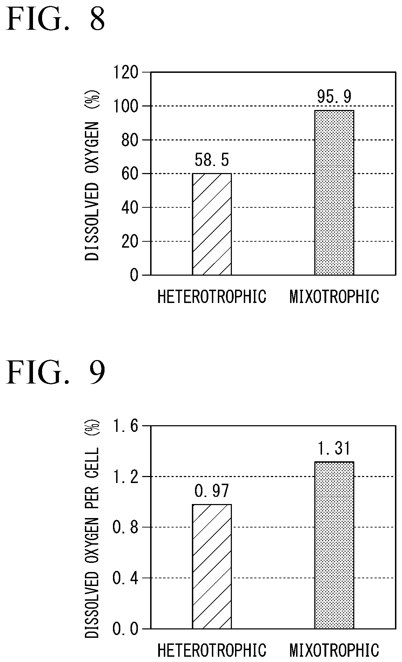

After the main culture for 3 days, the dissolved oxygen concentration of the culture solution was measured using a dissolved oxygen meter (InPro 6000 Amperometric O2 Sensors; METTLER TOLEDO, Switzerland) that had been subjected to two-point calibration. The results are shown in FIG. 8. From the results of FIG. 8, it was confirmed that, in the mixotrophic culture, the dissolved oxygen concentration of the culture solution was higher than that in the heterotrophic culture.

The dissolved oxygen concentration per cell was calculated by the following equation. The results are shown in FIG. 9.

Dissolved oxygen concentration per cell = dissolved oxygen concentration of culture solution/maximum cell density achieved

From the results of FIG. 9, it was confirmed that, in the mixotrophic culture, the dissolved oxygen concentration per cell was higher than that in the heterotrophic culture.

From the above results, it was considered that, in the mixotrophic culture, the dissolved oxygen concentration in the culture medium was increased by the oxygen generation by photosynthesis. It was considered that, as the dissolved oxygen concentration in the culture medium increased, the cell density of the algae increased. As a result, it was considered that the proliferation rate of the algae per unit culture time increased.

[Example 7]

(Component analysis)

A high-density growth strain (hereinafter, referred to as "SAG 108.79 n-1 strain") of a haploid of SAG 108.79 was obtained by the same operation as (productoin of haploid cells) and (production of high-density growth strain) in Example 1, except that Galdieria sulphuraria SAG 108.79 (diploid) was used instead of Galdieria partita NBRC 102759 (diploid) used in Example 1. Using this, mixotrophic culture and heterotrophic culture were performed. The algal cells were collected in a state where the $OD_{750}$ after the culture was 40 to 60, and the component analysis was performed by Japan Food Research Laboratories.

<Culture conditions>

Culture medium used: 3×MA + 300 mM sucrose (pH 1.5)
Amount of culture medium: 50 L
Illumination condition: 300 to 1,370 $\mu$mol photons/m$^2$s (mixotrophic culture, difference in light intensity occurs depending on the portion exposed to light) or dark (heterotrophic culture)
Temperature condition: 41°C
$CO_2$ condition: air was blown into the culture solution by an air pump ($CO_2$ concentration corresponds to $CO_2$ concentration in air)
Shaking speed: stirred by blowing air
Culture start $OD_{750}$: 1.5

The analysis results of the algal cells cultured by heterotrophic culture are shown in Tables 12 and 13. The analysis results of the algal cells cultured by mixotrophic culture are shown in Tables 14 and 15.

[Table 12]

| Heterotrophic culture of SAG 108.79 n-1 strain | | | | |
|---|---|---|---|---|
| Analytical item | Result | Limit of quantitation | Note | Method |
| Water | 16.0 g/100 g | | | Atmospheric-pressure heat drying method |
| Protein | 34.5 g/100 g | | 1 | Combustion method |
| Lipid | 4.8 g/100 g | | | Acid digestion method |
| Ash | 2.6 g/100 g | | | Direct ashing method |
| Total carbohydrate | 42.1 g/100 g | | 2 | |
| Available carbohydrate | 38.9 g/100 g | | 3 | |
| Dietary fiber | 3.2 g/100 g | | | Oxygen gravimetric method |
| Energy | 343 kcal/100 g | | 4 | |
| Sodium | 93.8 mg/100 g | | | Atomic absorption spectrophotometry |
| Salt equivalent | 0.238 g/100 g | | 5 | |
| Vitamin A (Retinol activity equivalents) | 821 g/100 g | | 6 | |

(continued)

| Heterotrophic culture of SAG 108.79 n-1 strain | | | | |
|---|---|---|---|---|
| Analytical item | Result | Limit of quantitation | Note | Method |
| α-carotene | Not detected | 50 g/100 g | | High-performance liquid chromatography |
| β-carotene | 9,850 g/100 g | | 7 | High-performance liquid chromatography |
| Total ascorbic acid (total vitamin C) | 38 mg/100 g | | 8 | High-performance liquid chromatography |
| Vitamin E (α-tocopherol) | 2.0 mg/100 g | | | High-performance liquid chromatography |
| Folate | 1,100 g/100 g | | 9 | Microbiological quantification method |

[Table 13]

| Heterotrophic culture of SAG 108.79 n-1 strain | | | |
|---|---|---|---|
| Analytical item | Result | Note | Method |
| Amino acid | | | |
| Arginine | 1.56 g/100 g | | Automated amino acid analysis |
| Lysine | 1.71 g/100 g | | Automated amino acid analysis |
| Histidine | 0.54 g/100 g | | Automated amino acid analysis |
| Phenylalanine | 1.23 g/100 g | | Automated amino acid analysis |
| Tyrosine | 1.02 g/100 g | | Automated amino acid analysis |
| Leucine | 2.13 g/100 g | | Automated amino acid analysis |
| Isoleucine | 1.23 g/100 g | | Automated amino acid analysis |
| Methionine | 0.56 g/100 g | 10 | Automated amino acid analysis |
| Valine | 1.37 g/100 g | | Automated amino acid analysis |
| Alanine | 1.34 g/100 g | | Automated amino acid analysis |
| Glycine | 1.14 g/100 g | | Automated amino acid analysis |
| Proline | 2.04 g/100 g | | Automated amino acid analysis |
| Glutamine | 2.97 g/100 g | | Automated amino acid analysis |
| Serine | 1.30 g/100 g | | Automated amino acid analysis |
| Threonine | 1.08 g/100 g | | Automated amino acid analysis |
| Aspartic acid | 2.31 g/100 g | | Automated amino acid analysis |
| Tryptophan | 0.39 g/100 g | | High-performance liquid chromatography |
| Cystine | 0.38 g/100 g | 10 | Automated amino acid analysis |
| Taurine | 0.10 g/100 g | | Automated amino acid analysis |
| Free γ-aminobutyric acid | 263 mg/100 g | | Automated amino acid analysis |

[Table 14]

| Mixotrophic culture of SAG 108.79 n-1 strain | | | | |
|---|---|---|---|---|
| Analytical item | Result | Limit of quantitation | Note | Method |
| Water | 14.2 g/100 g | | | Atmospheric-pressure heat drying method |
| Protein | 37.8 g/100 g | | 1 | Combustion method |
| Lipid | 4.8 g/100 g | | | Acid digestion method |
| Ash | 3.3 g/100 g | | | Direct ashing method |
| Total carbohydrate | 39.9 g/100 g | | 2 | |
| Available carbohydrate | 35.1 g/100 g | | 3 | |
| Dietary fiber | 4.8 g/100 g | | | Oxygen gravimetric method |
| Energy | 344 kcal/100 | | 4 | |
| Sodium | 94.4 mg/100 g | | | Atomic absorption spectrophotometry |
| Salt equivalent | 0.240 g/100 g | | 5 | |
| Vitamin A (Retinol activity equivalents) | 373 g/100 g | | 6 | |
| $\alpha$-carotene | Not detected | 10 g/100 g | | High-performance liquid |
| $\beta$-carotene | 4,480 g/100 g | | 7 | High-performance liquid chromatography |
| Total ascorbic acid (total vitamin C) | 57 mg/100 g | | 8 | High-performance liquid chroma-tography |
| Vitamin E ($\alpha$-tocopherol) | 1.2 mg/100 g | | | High-performance liquid chromatography |
| Folate | 1,400 g/100 g | | 9 | Microbiological quantification method |

[Table 15]

| Mixotrophic culture of SAG 108.79 n-1 strain | | | |
|---|---|---|---|
| Analytical item | Result | Note | Method |
| Amino acid | | | |
| Arginine | 1.69 g/100 g | | Automated amino acid analysis |
| Lysine | 1.86 g/100 g | | Automated amino acid analysis |
| Histidine | 0.57 g/100 g | | Automated amino acid analysis |
| Phenylalanine | 1.34 g/100 g | | Automated amino acid analysis |
| Tyrosine | 1.12 g/100 g | | Automated amino acid analysis |
| Leucine | 2.31 g/100 g | | Automated amino acid analysis |
| Isoleucine | 1.31 g/100 g | | Automated amino acid analysis |
| Methionine | 0.62 g/100 g | 10 | Automated amino acid analysis |
| Valine | 1.48 g/100 g | | Automated amino acid analysis |
| Alanine | 1.44 g/100 g | | Automated amino acid analysis |
| Glycine | 1.24 g/100 g | | Automated amino acid analysis |
| Proline | 1.83 g/100 g | | Automated amino acid analysis |

(continued)

| Mixotrophic culture of SAG 108.79 n-1 strain | | | |
|---|---|---|---|
| Analytical item | Result | Note | Method |
| Glutamine | 3.55 g/100 g | | Automated amino acid analysis |
| Serine | 1.46 g/100 g | | Automated amino acid analysis |
| Threonine | 1.19 g/100 g | | Automated amino acid analysis |
| Aspartic acid | 2.59 g/100 g | | Automated amino acid analysis |
| Tryptophan | 0.37 g/100 g | | High-performance liquid chromatography |
| Cystine | 0.42 g/100 g | 10 | Automated amino acid analysis |
| Taurine | 0.12 g/100 g | | Automated amino acid analysis |
| Free $\gamma$-aminobutyric acid | 143 mg/100 g | | Automated amino acid analysis |

The "Note" in Tables 12 to 15 is as follows.

1 Nitrogen-protein conversion factor: 6.25
2 Formula for calculation according to Japanese Food Labeling Standards (Cabinet Office Ordinance No. 10, 2015): 100 - (water + protein + lipid + ash)
3 Formula for calculation according to Japanese Food Labeling Standards (Cabinet Office Ordinance No. 10, 2015): 100 - (water + protein + lipid + ash + dietary fiber)
4 Energy conversion factor according to Japanese Food Labeling Standards (Cabinet Office Ordinance No. 10, 2015): protein, 4; lipid, 9; available carbohydrate, 4; dietary fiber, 2
5 Formula for calculation: sodium $\times$ 2.54
6 24 $\mu$g of $\alpha$-carotene and 12 $\mu$g of $\beta$-carotene were each converted into 1 $\mu$g of retinol activity equivalent.
7 On Japanese Food Labeling Standards (2015 Ministry of Health, Labour and Welfare Notice No. 139), annex for analysis method of nutritional components and the like
8 After derivatization with hydrazine, measurement was performed.
9 Strain used: Lactobacillus rhamnosus (L. casei) ATCC 7469
10 After formic acid oxidation treatment, hydrochloric acid hydrolysis and measurement were performed.

In the mixotrophic culture, the contents of total ascorbic acid and folate were higher than those in the heterotrophic culture.

Hereinabove, preferable examples of the present invention have been described above, but the present invention is not limited to these examples. Configurations can be added, omitted, and replaced, and other modifications can be made within a range not departing from the gist of the present invention. The present invention is not limited by the above description, but only by the scope of the appended claims.

## Claims

1. A method for high-density culture of unicellular algae, the method comprising:

   performing mixotrophic culture of unicellular algae,
   wherein the unicellular algae are algae that are capable of growing in a culture medium containing 0.1% by mass or more of sugars and having acidic conditions at a pH of 5 or less, and
   a chlorophyll content of the unicellular algae after culture for 7 days under dark conditions in the presence of an organic carbon source is 1 $\mu$g/mg dry weight or more.

2. The method for high-density culture of unicellular algae according to claim 1,
   wherein the unicellular algae are haploids of algae belonging to the class Cyanidiophyceae.

3. The method for high-density culture of unicellular algae according to claim 1,
   wherein the unicellular algae are homozygous diploids produced from haploids of algae belonging to the class Cyanidiophyceae.

4. A method for producing unicellular algae, the method comprising:

(ia) a step of producing haploid unicellular algae from diploid unicellular algae;
(iia) a step of seeding the haploid unicellular algae on a solid culture medium containing an organic carbon source and culturing the seeded haploid unicellular algae under dark conditions; and
(iiia) a step of collecting a green colony from colonies that have appeared on the solid culture medium.

5. The method for producing unicellular algae according to claim 4,
wherein the unicellular algae are algae belonging to the class Cyanidiophyceae.

6. A method for producing unicellular algae, the method comprising:

(ib) a step of producing diploid unicellular algae from haploid unicellular algae;
(iib) a step of seeding the diploid unicellular algae on a solid culture medium containing an organic carbon source and culturing the seeded diploid unicellular algae under dark conditions; and
(iiib) a step of collecting a green colony from colonies that have appeared on the solid culture medium.

7. The method for producing unicellular algae according to claim 6,
wherein the unicellular algae are algae belonging to the class Cyanidiophyceae.

8. Unicellular algae that are capable of growing in a culture medium containing 0.1% by mass or more of sugars and having acidic conditions at a pH of 5 or less,
wherein a chlorophyll content of the unicellular algae after culture for 7 days under dark conditions in the presence of an organic carbon source is 1 $\mu$g/mg dry weight or more.

9. The unicellular algae according to claim 8,
wherein the unicellular algae are haploid algae belonging to the class Cyanidiophyceae.

10. The unicellular algae according to claim 8,
wherein the unicellular algae are homozygous diploid algae produced from haploid algae belonging to the class Cyanidiophyceae.

# FIG. 1

METABOLOME ANALYSIS (HYDROPHILIC METABOLITE-FOCUSED)
SAMPLE ADJUSTMENT METHOD
CONTRACTOR: KAZUSA DNA RESEARCH INSTITUTE

1. EXTRACTION

SAMPLE

←—DISRUPTION TREATMENT
←—METHANOL (FINAL CONCENTRATION OF 70% TO 80%)

HOMOGENIZATION

CENTRIFUGATION

COLLECTION OF SUPERNATANT

2. PURIFICATION

MonoSpin C18 COLUMN (GL Science)

←—CONDITIONING ACCORDING TO PRODUCT MANUAL
←—LOADING OF EXTRACTED SAMPLE

COLLECTION OF COLUMN FLOW-THROUGH FRACTION

3. DERIVATIZATION

COLUMN FLOW-THROUGH FRACTION

←—DRYING (NITROGEN GAS BLOWING OR CENTRIFUGAL EVAPORATOR)
←—ADDITION OF METHOXYAMINE/PYRIDINE

METHOXYMATION

←—ADDITION OF N-METHYL-N-(TRIMETHYLSILYL)TRIFLUOROACETAMIDE

TRIMETHYLSILYLATION

FIG. 2

FIG. 3

# FIG. 4

# FIG. 5

# FIG. 6

# FIG. 7

FIG. 8

FIG. 9

## INTERNATIONAL SEARCH REPORT

| International application No. |
| --- |
| **PCT/JP2024/029913** |

| A. | CLASSIFICATION OF SUBJECT MATTER |
| --- | --- |

*C12N 1/12*(2006.01)i
FI:   C12N1/12 A; C12N1/12 C

According to International Patent Classification (IPC) or to both national classification and IPC

| B. | FIELDS SEARCHED |
| --- | --- |

Minimum documentation searched (classification system followed by classification symbols)

C12N1/12-1/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); PubMed

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | HIROOKA, S. et al. Life cycle and functional genomics of the unicellular red alga Galdieria for elucidating algal and plant evolution and industrial use. PNAS. 2022, vol. 119, no. 41, e2210665119, pp. 1-12 <br> Results and Discussion, figures | 1-10 |
| A | WO 2019/107385 A1 (JAPAN SCIENCE AND TECHNOLOGY AGENCY) 06 June 2019 (2019-06-06) <br> claims, examples | 1-10 |
| A | JP 2008-17718 A (INSTITUTE OF PHYSICAL & CHEMICAL RESEARCH) 31 January 2008 (2008-01-31) <br> claims, examples | 1-10 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 October 2024** | **29 October 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2024/029913**

| Patent document cited in search report | | | Publication date (day/month/year) | Patent family member(s) | | | Publication date (day/month/year) |
|---|---|---|---|---|---|---|---|
| WO | 2019/107385 | A1 | 06 June 2019 | US | 2021/0147791 | A1 | |
| | | | | claims, examples | | | |
| | | | | EP | 3719114 | A1 | |
| JP | 2008-17718 | A | 31 January 2008 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023134686 A

- WO 2019107385 A

**Non-patent literature cited in the description**

- **WOLFGANG GROSS** ; **CLAUS SCHNARRENBER-GER**. Heterotrophic Growth of Two Strains of the Acido-Thermophilic Red Alga Galdieria sulphuraria. *Plant Cell Physiol*, 1995, vol. 36 (4), 633-638
- Life cycle and functional genomics of the unicellular red alga Galdieria for elucidating algal and plant evolution and industrial use. *Proc Natl Acad Sci*, 2022, vol. 119 (41), e2210665119
- **A. R. WELLBURN**. *J. Plant Physiol.*, 1994, vol. 144, 307-313

- **SHAO-LUN LIU et al.** *J. Phycol.*, December 2020, vol. 56 (6), 1428-1442
- **ALLEN MB.** *Arch. Microbiol.*, 1959, vol. 32, 270-277
- **MINODA A et al.** *Plant Cell Physiol*, 2004, vol. 45, 667-71
- **OHNUMA M et al.** *Plant Cell Physiol.*, January 2008, vol. 49 (1), 7-20